# EUROPEAN PATENT SPECIFICATION

(11) **EP 2 817 623 B1**
(45) Date of publication and mention of the grant of the patent: **08.05.2019**
(21) Application number: 13711297.5
(22) Date of filing: 25.02.2013
(51) Int. Cl.: G01N 33/564

(54) **IDENTIFICATION OF BINDING PROPERTIES OF ANTIBODIES REACTIVE WITH A MEMBER OF THE INSULIN RECEPTOR FAMILY**
IDENTIFIZIERUNG DER BINDUNGSEIGENSCHAFTEN VON MIT EINEM MITGLIED DER INSULINREZEPTORFAMILIE REAKTIVEN ANTIKÖRPERN
IDENTIFICATION DES PROPRIÉTÉS DE LIAISON D'ANTICORPS RÉAGISSANT AVEC UN MEMBRE DE LA FAMILLE DES RÉCEPTEURS D'INSULINE

(30) Priority: 24.02.2012 EP 12156930
(43) Date of publication of application: 31.12.2014
(60) Divisional application: 19156858.3
(73) Proprietor: berYsol GmbH, 10965 Berlin (DE)
(72) Inventor: SCHOMBURG, Lutz, 10965 Berlin (DE); MINICH, Waldemar, 13599 Berlin (DE); KARASCH, Siegmund, 12355 Berlin (DE)
(86) International application number: PCT/EP2013/053707
(87) International publication number: WO 2013/124482

(56) References cited:
- EP-A2- 0 313 986
- WO-A1-2006/097521
- WO-A1-2006/097521
- WO-A1-2011/139681
- CN-A- 101 968 483
- COHEN B ET AL: "Combination therapy enhances the inhibition of tumor growth with the fully human anti-type 1 insulin-like growth factor receptor monoclonal antibody CP-751,871", CLINICAL CANCER RESEARCH, THE AMERICAN ASSOCIATION FOR CANCER RESEARCH, US, vol. 11, no. 5, 1 March 2005 (2005-03-01), pages 2063-2073, XP002433667, ISSN: 1078-0432, DOI: 10.1158/1078-0432.CCR-04-1070
- KATHLEEN THOMPSON ET AL: "Low Prevalence of Autoantibodies to the Insulin-like Growth Factor I Receptor in Children with Short Stature", PEDIATRIC RESEARCH, vol. 32, no. 4, 1 October 1992 (1992-10-01), pages 455-459, XP055035993, ISSN: 0031-3998, DOI: 10.1203/00006450-199210000-00016
- A L DANIEL: "Type B Insulin Resistance Developing during Interferon alpha Therapy", ENDOCR PRACT, vol. 15, no. 2, 1 January 2009 (2009-01-01), pages 153-157, XP055036018,
- S.-X. ZHAO ET AL: "Orbital fibrosis in a mouse model of Graves' disease induced by genetic immunization of thyrotropin receptor cDNA", JOURNAL OF ENDOCRINOLOGY, vol. 210, no. 3, 1 September 2011 (2011-09-01), pages 369-377, XP055035869, ISSN: 0022-0795, DOI: 10.1530/JOE-11-0162
- RODRIGUEZ O ET AL: "Characterization of purified autoantibodies to the insulin receptor from six patients with type B insulin resistance", METABOLISM, CLINICAL AND EXPERIMENTAL, W.B. SAUNDERS CO., PHILADELPHIA, PA, US, vol. 41, no. 3, 1 March 1992 (1992-03-01), pages 325-331, XP026314260, ISSN: 0026-0495, DOI: 10.1016/0026-0495(92)90279-J [retrieved on 1992-03-01]
- B. ZHANG ET AL: "A region of the insulin receptor important for ligand binding (residues 450-601) is recognized by patients' autoimmune antibodies and inhibitory monoclonal antibodies.", PROCEEDINGS OF THE NATIONAL ACADEMY OF SCIENCES, vol. 88, no. 21, 1 November 1991 (1991-11-01), pages 9858-9862, XP055035879, ISSN: 0027-8424, DOI: 10.1073/pnas.88.21.9858
- ARIOGLU ELIF ET AL: "Clinical course of the syndrome of autoantibodies to the insulin receptor (type B insulin resistance): a 28-year perspective", MEDICINE, WILLIAMS AND WILKINS, BALTIMORE, US, vol. 81, no. 2, 1 March 2002 (2002-03-01), pages 87-100, XP009162083, ISSN: 0025-7974
- DI PAOLO S ET AL: "Insulin resistance and hypoglycemia in a patient with systemic lupus erythematosus: description of antiinsulin receptor antibodies that enhance insulin binding and inhibit insulin action", JOURNAL OF CLINICAL ENDOCRINOLOGY AND METABOLISM, THE ENDOCRINE SOCIETY, US, vol. 73, no. 3, 1 September 1991 (1991-09-01), pages 650-657, XP009162084, ISSN: 0021-972X
- PANDINI ET AL: "Functional responses and in vivo anti-tumour activity of h7C10: A humanised monoclonal antibody with neutralising activity against the insulin-like growth factor-1 (IGF-1) receptor and insulin/IGF-1 hybrid receptors", EUROPEAN JOURNAL OF CANCER, PERGAMON PRESS, OXFORD, GB, vol. 43, no. 8, 1 May 2007 (2007-05-01), pages 1318-1327, XP022072243, ISSN: 0959-8049, DOI: 10.1016/J.EJCA.2007.03.009
- COHEN B ET AL: "Combination therapy enhances the inhibition of tumor growth with the fully human anti-type 1 insulin-like growth factor receptor monoclonal antibody CP-751,871", CLINICAL CANCER RESEARCH, THE AMERICAN ASSOCIATION FOR CANCER RESEARCH, US, vol. 11, no. 5, 1 March 2005 (2005-03-01), pages 2063-2073, XP002433667, ISSN: 1078-0432, DOI: 10.1158/1078-0432.CCR-04-1070
- MASSEYEFFEF(EDITOR-IN-CHIEF) ED - ALBERT W H ET AL: "Methods of Immunological Analysis, Vol. 1, Fundamentals, passage", 1 January 1993 (1993-01-01), METHODS OF IMMUNOLOGICAL ANALYSIS. FUNDAMENTALS; [METHODS OF IMMUNOLOGICAL ANALYSIS], WEINHEIM, VCH, DE, PAGE(S) I - III,475, XP002325263,
- YIN KUO-CHANG ET AL: "Evaluation of two ELISA methods to detect therapeutic anti-IGF1R antibodies in clinical study samples of dalotuzumab", BIOANALYSIS, FUTURE SCIENCE, UK, vol. 3, no. 18, 1 September 2011 (2011-09-01), pages 2107-2117, XP009162082, ISSN: 1757-6199, DOI: 10.4155/BIO.11.208

### SEQUENCE LISTING

<110> Charite - Universitaetsmedizin Berlin ICI immunochemical intelligence GmbH
<120> IDENTIFICATION OF MODULATORS OF BINDING PROPERTIES OF ANTIBODIES
   REACTIVE WITH A MEMBER OF THE INSULIN RECEPTOR FAMILY
<130> DG011022-P
<150> EP12156930.5
   <151> 2012-02-24
<160> 20
<170> BiSSAP 1.2
<210> 1
   <211> 33
   <212> DNA
   <213> Artificial Sequence
<220>
   <221> source
   <222> 1..33
   <223> /organism="Artificial Sequence" /note="primer P1" /mol_type="unassigned DNA"
<400> 1
   gcggaattcg tcaccgacgc caaaaacata aag 33
<210> 2
   <211> 34
   <212> DNA
   <213> Artificial Sequence
<220>
   <221> source
   <222> 1..34
   <223> /organism="Artificial Sequence" /note="Primer P2" /mol_type="unassigned DNA"
<400> 2
   acgggattct tacacggcga tctttccgcc cttc 34
<210> 3
   <211> 35
   <212> DNA
   <213> Artificial Sequence
<220>
   <221> source
   <222> 1..35
   <223> /organism="Artificial Sequence" /note="Primer P3" /mol_type="unassigned DNA"
<400> 3
   acggcggccg catgaagtct ggctccggag gaggg 35
<210> 4
   <211> 33
   <212> DNA
   <213> Artificial Sequence
<220>
   <221> source
   <222> 1..33
   <223> /organism="Artificial Sequence" /note="Primer P4" /mol_type="unassigned DNA"
<400> 4
   acggaattcg caggtcgaag actggggcag cgg 33
<210> 5
   <211> 1653
   <212> DNA
   <213> Photinus pyralis
<220>
   <221> source
   <222> 1..1653
   <223> /organism="Photinus pyralis" /note="cds" /mol_type="unassigned DNA"
<400> 5
   gtcaccgacg ccaaaaacat aaagaaaggc ccggcgccat tctatccgct ggaagatgga      60
   accgctggag agcaactgca taaggctatg aagagatacg ccctggttcc tggaacaatt     120
   gcttttacag atgcacatat cgaggtggac atcacttacg ctgagtactt cgaaatgtcc     180
   gttcggttgg cagaagctat gaaacgatat gggctgaata caaatcacag aatcgtcgta     240
   tgcagtgaaa actctcttca attctttatg ccggtgttgg gcgcgttatt tatcggagtt     300
   gcagttgcgc ccgcgaacga catttataat gaacgtgaat tgctcaacag tatgggcatt     360
   tcgcagccta ccgtggtgtt cgtttccaaa aaggggttgc aaaaaatttt gaacgtgcaa     420
   aaaaagctcc caatcatcca aaaaattatt atcatggatt ctaaaacgga ttaccaggga     480
   tttcagtcga tgtacacgtt cgtcacatct catctacctc ccggttttaa tgaatacgat     540
   tttgtgccag agtccttcga tagggacaag acaattgcac tgatcatgaa ctcctctgga     600
   tctactggtc tgcctaaagg tgtcgctctg cctcatagaa ctgcctgcgt gagattctcg     660
   catgccagag atcctatttt tggcaatcaa atcattccgg atactgcgat tttaagtgtt     720
   gttccattcc atcacggttt tggaatgttt actacactcg gatatttgat atgtggattt     780
   cgagtcgtct taatgtatag atttgaagaa gagctgtttc tgaggagcct tcaggattac     840
   aagattcaaa gtgcgctgct ggtgccaacc ctattctcct tcttcgccaa aagcactctg     900
   attgacaaat acgatttatc taatttacac gaaattgctt ctggtggcgc tcccctctct     960
   aaggaagtcg gggaagcggt tgccaagagg ttccatctgc caggtatcag gcaaggatat    1020
   gggctcactg agactacatc agctattctg attacacccg agggggatga taaaccgggc    1080
   gcggtcggta aagttgttcc attttttgaa gcgaaggttg tggatctgga taccgggaaa    1140
   acgctgggcg ttaatcaaag aggcgaactg tgtgtgagag gtcctatgat tatgtccggt    1200
   tatgtaaaca atccggaagc gaccaacgcc ttgattgaca aggatggatg gctacattct    1260
   ggagacatag cttactggga cgaagacgaa cacttcttca tcgttgaccg cctgaagtct    1320
   ctgattaagt acaaaggcta tcaggtggct cccgctgaat tggaatccat cttgctccaa    1380
   caccccaaca tcttcgacgc aggtgtcgca ggtcttcccg acgatgacgc cggtgaactt    1440
   cccgccgccg ttgttgtttt ggagcacgga aagacgatga cggaaaaaga gatcgtggat    1500
   tacgtcgcca gtcaagtaac aaccgcgaaa aagttgcgcg gaggagttgt gtttgtggac    1560
   gaagtaccga aaggtcttac cggaaaactc gacgcaagaa aaatcagaga gatcctcata    1620
   aaggccaaga agggcggaaa gatcgccgtg taa                                 1653
<210> 6
   <211> 550
   <212> PRT
   <213> Photinus pyralis
<400> 6
   Val Thr Asp Ala Lys Asn Ile Lys Lys Gly Pro Ala Pro Phe Tyr Pro
   1               5                   10                  15
   Leu Glu Asp Gly Thr Ala Gly Glu Gln Leu His Lys Ala Met Lys Arg
               20                  25                  30
   Tyr Ala Leu Val Pro Gly Thr Ile Ala Phe Thr Asp Ala His Ile Glu
           35                  40                  45
   Val Asp Ile Thr Tyr Ala Glu Tyr Phe Glu Met Ser Val Arg Leu Ala
       50                  55                  60
   Glu Ala Met Lys Arg Tyr Gly Leu Asn Thr Asn His Arg Ile Val Val
   65                  70                  75                  80
   Cys Ser Glu Asn Ser Leu Gln Phe Phe Met Pro Val Leu Gly Ala Leu
                   85                  90                  95
   Phe Ile Gly Val Ala Val Ala Pro Ala Asn Asp Ile Tyr Asn Glu Arg
               100                 105                 110
   Glu Leu Leu Asn Ser Met Gly Ile Ser Gln Pro Thr Val Val Phe Val
           115                 120                 125
   Ser Lys Lys Gly Leu Gln Lys Ile Leu Asn Val Gln Lys Lys Leu Pro
       130                 135                 140
   Ile Ile Gln Lys Ile Ile Ile Met Asp Ser Lys Thr Asp Tyr Gln Gly
   145                 150                 155                 160
   Phe Gln Ser Met Tyr Thr Phe Val Thr Ser His Leu Pro Pro Gly Phe
                   165                 170                 175
   Asn Glu Tyr Asp Phe Val Pro Glu Ser Phe Asp Arg Asp Lys Thr Ile
               180                 185                 190
   Ala Leu Ile Met Asn Ser Ser Gly Ser Thr Gly Leu Pro Lys Gly Val
           195                 200                 205
   Ala Leu Pro His Arg Thr Ala Cys Val Arg Phe Ser His Ala Arg Asp
       210                 215                 220
   Pro Ile Phe Gly Asn Gln Ile Ile Pro Asp Thr Ala Ile Leu Ser Val
   225                 230                 235                 240
   Val Pro Phe His His Gly Phe Gly Met Phe Thr Thr Leu Gly Tyr Leu
                   245                 250                 255
   Ile Cys Gly Phe Arg Val Val Leu Met Tyr Arg Phe Glu Glu Glu Leu
               260                 265                 270
   Phe Leu Arg Ser Leu Gln Asp Tyr Lys Ile Gln Ser Ala Leu Leu Val
           275                 280                 285
   Pro Thr Leu Phe Ser Phe Phe Ala Lys Ser Thr Leu Ile Asp Lys Tyr
       290                 295                 300
   Asp Leu Ser Asn Leu His Glu Ile Ala Ser Gly Gly Ala Pro Leu Ser
   305                 310                 315                 320
   Lys Glu Val Gly Glu Ala Val Ala Lys Arg Phe His Leu Pro Gly Ile
                   325                 330                 335
   Arg Gln Gly Tyr Gly Leu Thr Glu Thr Thr Ser Ala Ile Leu Ile Thr
               340                 345                 350
   Pro Glu Gly Asp Asp Lys Pro Gly Ala Val Gly Lys Val Val Pro Phe
           355                 360                 365
   Phe Glu Ala Lys Val Val Asp Leu Asp Thr Gly Lys Thr Leu Gly Val
       370                 375                 380
   Asn Gln Arg Gly Glu Leu Cys Val Arg Gly Pro Met Ile Met Ser Gly
   385                 390                 395                 400
   Tyr Val Asn Asn Pro Glu Ala Thr Asn Ala Leu Ile Asp Lys Asp Gly
                   405                 410                 415
   Trp Leu His Ser Gly Asp Ile Ala Tyr Trp Asp Glu Asp Glu His Phe
               420                 425                 430
   Phe Ile Val Asp Arg Leu Lys Ser Leu Ile Lys Tyr Lys Gly Tyr Gln
           435                 440                 445
   Val Ala Pro Ala Glu Leu Glu Ser Ile Leu Leu Gln His Pro Asn Ile
       450                 455                 460
   Phe Asp Ala Gly Val Ala Gly Leu Pro Asp Asp Asp Ala Gly Glu Leu
   465                 470                 475                 480
   Pro Ala Ala Val Val Val Leu Glu His Gly Lys Thr Met Thr Glu Lys
                   485                 490                 495
   Glu Ile Val Asp Tyr Val Ala Ser Gln Val Thr Thr Ala Lys Lys Leu
               500                 505                 510
   Arg Gly Gly Val Val Phe Val Asp Glu Val Pro Lys Gly Leu Thr Gly
           515                 520                 525
   Lys Leu Asp Ala Arg Lys Ile Arg Glu Ile Leu Ile Lys Ala Lys Lys
       530                 535                 540
   Gly Gly Lys Ile Ala Val
   545                 550
<210> 7
   <211> 4104
   <212> DNA
   <213> Homo sapiens
<220>
   <221> source
   <222> 1..4104
   <223> /organism="Homo sapiens" /note="IGF1R" /mol_type="unassigned DNA"
<400> 7
   atgaagtctg gctccggagg agggtccccg acctcgctgt gggggctcct gtttctctcc     60
   gccgcgctct cgctctggcc gacgagtgga gaaatctgcg ggccaggcat cgacatccgc    120
   aacgactatc agcagctgaa gcgcctggag aactgcacgg tgatcgaggg ctacctccac    180
   atcctgctca tctccaaggc cgaggactac cgcagctacc gcttccccaa gctcacggtc    240
   attaccgagt acttgctgct gttccgagtg gctggcctcg agagcctcgg agacctcttc    300
   cccaacctca cggtcatccg cggctggaaa ctcttctaca actacgccct ggtcatcttc     360
   gagatgacca atctcaagga tattgggctt tacaacctga ggaacattac tcggggggcc    420
   atcaggattg agaaaaatgc tgacctctgt tacctctcca ctgtggactg gtccctgatc     480
   ctggatgcgg tgtccaataa ctacattgtg gggaataagc ccccaaagga atgtggggac     540
   ctgtgtccag ggaccatgga ggagaagccg atgtgtgaga agaccaccat caacaatgag     600
   tacaactacc gctgctggac cacaaaccgc tgccagaaaa tgtgcccaag cacgtgtggg     660
   aagcgggcgt gcaccgagaa caatgagtgc tgccaccccg agtgcctggg cagctgcagc     720
   gcgcctgaca acgacacggc ctgtgtagct tgccgccact actactatgc cggtgtctgt     780
   gtgcctgcct gcccgcccaa cacctacagg tttgagggct ggcgctgtgt ggaccgtgac     840
   ttctgcgcca acatcctcag cgccgagagc agcgactccg aggggtttgt gatccacgac     900
   ggcgagtgca tgcaggagtg cccctcgggc ttcatccgca acggcagcca gagcatgtac     960
   tgcatccctt gtgaaggtcc ttgcccgaag gtctgtgagg aagaaaagaa aacaaagacc    1020
   attgattctg ttacttctgc tcagatgctc caaggatgca ccatcttcaa gggcaatttg    1080
   ctcattaaca tccgacgggg gaataacatt gcttcagagc tggagaactt catggggctc    1140
   atcgaggtgg tgacgggcta cgtgaagatc cgccattctc atgccttggt ctccttgtcc    1200
   ttcctaaaaa accttcgcct catcctagga gaggagcagc tagaagggaa ttactccttc    1260
   tacgtcctcg acaaccagaa cttgcagcaa ctgtgggact gggaccaccg caacctgacc    1320
   atcaaagcag ggaaaatgta ctttgctttc aatcccaaat tatgtgtttc cgaaatttac    1380
   cgcatggagg aagtgacggg gactaaaggg cgccaaagca aaggggacat aaacaccagg    1440
   aacaacgggg agagagcctc ctgtgaaagt gacgtcctgc atttcacctc caccaccacg    1500
   tcgaagaatc gcatcatcat aacctggcac cggtaccggc cccctgacta cagggatctc    1560
   atcagcttca ccgtttacta caaggaagca ccctttaaga atgtcacaga gtatgatggg    1620
   caggatgcct gcggctccaa cagctggaac atggtggacg tggacctccc gcccaacaag    1680
   gacgtggagc ccggcatctt actacatggg ctgaagccct ggactcagta cgccgtttac    1740
   gtcaaggctg tgaccctcac catggtggag aacgaccata tccgtggggc caagagtgag    1800
   atcttgtaca ttcgcaccaa tgcttcagtt ccttccattc ccttggacgt tctttcagca    1860
   tcgaactcct cttctcagtt aatcgtgaag tggaaccctc cctctctgcc caacggcaac    1920
   ctgagttact acattgtgcg ctggcagcgg cagcctcagg acggctacct ttaccggcac    1980
   aattactgct ccaaagacaa aatccccatc aggaagtatg ccgacggcac catcgacatt    2040
   gaggaggtca cagagaaccc caagactgag gtgtgtggtg gggagaaagg gccttgctgc    2100
   gcctgcccca aaactgaagc cgagaagcag gccgagaagg aggaggctga ataccgcaaa    2160
   gtctttgaga atttcctgca caactccatc ttcgtgccca gacctgaaag gaagcggaga    2220
   gatgtcatgc aagtggccaa caccaccatg tccagccgaa gcaggaacac cacggccgca 2280
   gacacctaca acatcaccga cccggaagag ctggagacag agtacccttt ctttgagagc 2340
   agagtggata acaaggagag aactgtcatt tctaaccttc ggcctttcac attgtaccgc 2400
   atcgatatcc acagctgcaa ccacgaggct gagaagctgg gctgcagcgc ctccaacttc 2460
   gtctttgcaa ggactatgcc cgcagaagga gcagatgaca ttcctgggcc agtgacctgg 2520
   gagccaaggc ctgaaaactc catcttttta aagtggccgg aacctgagaa tcccaatgga 2580
   ttgattctaa tgtatgaaat aaaatacgga tcacaagttg aggatcagcg agaatgtgtg 2640
   tccagacagg aatacaggaa gtatggaggg gccaagctaa accggctaaa cccggggaac 2700
   tacacagccc ggattcaggc cacatctctc tctgggaatg ggtcgtggac agatcctgtg 2760
   ttcttctatg tccaggccaa aacaggatat gaaaacttca tccatctgat catcgctctg 2820
   cccgtcgctg tcctgttgat cgtgggaggg ttggtgatta tgctgtacgt cttccataga 2880
   aagagaaata acagcaggct ggggaatgga gtgctgtatg cctctgtgaa cccggagtac 2940
   ttcagcgctg ctgatgtgta cgttcctgat gagtgggagg tggctcggga gaagatcacc 3000
   atgagccggg aacttgggca ggggtcgttt gggatggtct atgaaggagt tgccaagggt 3060
   gtggtgaaag atgaacctga aaccagagtg gccattaaaa cagtgaacga ggccgcaagc 3120
   atgcgtgaga ggattgagtt tctcaacgaa gcttctgtga tgaaggagtt caattgtcac 3180
   catgtggtgc gattgctggg tgtggtgtcc caaggccagc caacactggt catcatggaa 3240
   ctgatgacac ggggcgatct caaaagttat ctccggtctc tgaggccaga aatggagaat 3300
   aatccagtcc tagcacctcc aagcctgagc aagatgattc agatggccgg agagattgca 3360
   gacggcatgg catacctcaa cgccaataag ttcgtccaca gagaccttgc tgcccggaat 3420
   tgcatggtag ccgaagattt cacagtcaaa atcggagatt ttggtatgac gcgagatatc 3480
   tatgagacag actattaccg gaaaggaggg aaagggctgc tgcccgtgcg ctggatgtct 3540
   cctgagtccc tcaaggatgg agtcttcacc acttactcgg acgtctggtc cttcggggtc 3600
   gtcctctggg agatcgccac actggccgag cagccctacc agggcttgtc caacgagcaa 3660
   gtccttcgct tcgtcatgga gggcggcctt ctggacaagc cagacaactg tcctgacatg 3720
   ctgtttgaac tgatgcgcat gtgctggcag tataacccca agatgaggcc ttccttcctg 3780
   gagatcatca gcagcatcaa agaggagatg gagcctggct tccgggaggt ctccttctac 3840
   tacagcgagg agaacaagct gcccgagccg gaggagctgg acctggagcc agagaacatg 3900
   gagagcgtcc ccctggaccc ctcggcctcc tcgtcctccc tgccactgcc cgacagacac 3960
   tcaggacaca aggccgagaa cggccccggc cctggggtgc tggtcctccg cgccagcttc 4020
   gacgagagac agccttacgc ccacatgaac gggggccgca agaacgagcg ggccttgccg 4080
   ctgccccagt cttcgacctg ctga 4104
<210> 8
   <211> 1367
   <212> PRT
   <213> Homo sapiens
<220>
   <223> IGF1R
<400> 8
<210> 9
   <211> 5760
   <212> DNA
   <213> Artificial Sequence
<220>
   <221> source
   <222> 1..5760
   <223> /organism="Artificial Sequence" /note="hIGF1R-luc fusion" /mol_type="unassigned DNA"
<400> 9
   atgaagtctg gctccggagg agggtccccg acctcgctgt gggggctcct gtttctctcc 60
   gccgcgctct cgctctggcc gacgagtgga gaaatctgcg ggccaggcat cgacatccgc 120
   aacgactatc agcagctgaa gcgcctggag aactgcacgg tgatcgaggg ctacctccac 180
   atcctgctca tctccaaggc cgaggactac cgcagctacc gcttccccaa gctcacggtc 240
   attaccgagt acttgctgct gttccgagtg gctggcctcg agagcctcgg agacctcttc 300
   cccaacctca cggtcatccg cggctggaaa ctcttctaca actacgccct ggtcatcttc 360
   gagatgacca atctcaagga tattgggctt tacaacctga ggaacattac tcggggggcc 420
   atcaggattg agaaaaatgc tgacctctgt tacctctcca ctgtggactg gtccctgatc 480
   ctggatgcgg tgtccaataa ctacattgtg gggaataagc ccccaaagga atgtggggac 540
   ctgtgtccag ggaccatgga ggagaagccg atgtgtgaga agaccaccat caacaatgag 600
   tacaactacc gctgctggac cacaaaccgc tgccagaaaa tgtgcccaag cacgtgtggg 660
   aagcgggcgt gcaccgagaa caatgagtgc tgccaccccg agtgcctggg cagctgcagc 720
   gcgcctgaca acgacacggc ctgtgtagct tgccgccact actactatgc cggtgtctgt 780
   gtgcctgcct gcccgcccaa cacctacagg tttgagggct ggcgctgtgt ggaccgtgac 840
   ttctgcgcca acatcctcag cgccgagagc agcgactccg aggggtttgt gatccacgac 900
   ggcgagtgca tgcaggagtg cccctcgggc ttcatccgca acggcagcca gagcatgtac 960
   tgcatccctt gtgaaggtcc ttgcccgaag gtctgtgagg aagaaaagaa aacaaagacc 1020
   attgattctg ttacttctgc tcagatgctc caaggatgca ccatcttcaa gggcaatttg 1080
   ctcattaaca tccgacgggg gaataacatt gcttcagagc tggagaactt catggggctc 1140
   atcgaggtgg tgacgggcta cgtgaagatc cgccattctc atgccttggt ctccttgtcc 1200
   ttcctaaaaa accttcgcct catcctagga gaggagcagc tagaagggaa ttactccttc 1260
   tacgtcctcg acaaccagaa cttgcagcaa ctgtgggact gggaccaccg caacctgacc 1320
   atcaaagcag ggaaaatgta ctttgctttc aatcccaaat tatgtgtttc cgaaatttac 1380
   cgcatggagg aagtgacggg gactaaaggg cgccaaagca aaggggacat aaacaccagg 1440
   aacaacgggg agagagcctc ctgtgaaagt gacgtcctgc atttcacctc caccaccacg 1500
   tcgaagaatc gcatcatcat aacctggcac cggtaccggc cccctgacta cagggatctc 1560
   atcagcttca ccgtttacta caaggaagca ccctttaaga atgtcacaga gtatgatggg 1620
   caggatgcct gcggctccaa cagctggaac atggtggacg tggacctccc gcccaacaag 1680
   gacgtggagc ccggcatctt actacatggg ctgaagccct ggactcagta cgccgtttac 1740
   gtcaaggctg tgaccctcac catggtggag aacgaccata tccgtggggc caagagtgag 1800
   atcttgtaca ttcgcaccaa tgcttcagtt ccttccattc ccttggacgt tctttcagca 1860
   tcgaactcct cttctcagtt aatcgtgaag tggaaccctc cctctctgcc caacggcaac 1920
   ctgagttact acattgtgcg ctggcagcgg cagcctcagg acggctacct ttaccggcac 1980
   aattactgct ccaaagacaa aatccccatc aggaagtatg ccgacggcac catcgacatt 2040
   gaggaggtca cagagaaccc caagactgag gtgtgtggtg gggagaaagg gccttgctgc 2100
   gcctgcccca aaactgaagc cgagaagcag gccgagaagg aggaggctga ataccgcaaa 2160
   gtctttgaga atttcctgca caactccatc ttcgtgccca gacctgaaag gaagcggaga 2220
   gatgtcatgc aagtggccaa caccaccatg tccagccgaa gcaggaacac cacggccgca 2280
   gacacctaca acatcaccga cccggaagag ctggagacag agtacccttt ctttgagagc 2340
   agagtggata acaaggagag aactgtcatt tctaaccttc ggcctttcac attgtaccgc 2400
   atcgatatcc acagctgcaa ccacgaggct gagaagctgg gctgcagcgc ctccaacttc 2460
   gtctttgcaa ggactatgcc cgcagaagga gcagatgaca ttcctgggcc agtgacctgg 2520
   gagccaaggc ctgaaaactc catcttttta aagtggccgg aacctgagaa tcccaatgga 2580
   ttgattctaa tgtatgaaat aaaatacgga tcacaagttg aggatcagcg agaatgtgtg 2640
   tccagacagg aatacaggaa gtatggaggg gccaagctaa accggctaaa cccggggaac 2700
   tacacagccc ggattcaggc cacatctctc tctgggaatg ggtcgtggac agatcctgtg 2760
   ttcttctatg tccaggccaa aacaggatat gaaaacttca tccatctgat catcgctctg 2820
   cccgtcgctg tcctgttgat cgtgggaggg ttggtgatta tgctgtacgt cttccataga 2880
   aagagaaata acagcaggct ggggaatgga gtgctgtatg cctctgtgaa cccggagtac 2940
   ttcagcgctg ctgatgtgta cgttcctgat gagtgggagg tggctcggga gaagatcacc 3000
   atgagccggg aacttgggca ggggtcgttt gggatggtct atgaaggagt tgccaagggt 3060
   gtggtgaaag atgaacctga aaccagagtg gccattaaaa cagtgaacga ggccgcaagc 3120
   atgcgtgaga ggattgagtt tctcaacgaa gcttctgtga tgaaggagtt caattgtcac 3180
   catgtggtgc gattgctggg tgtggtgtcc caaggccagc caacactggt catcatggaa 3240
   ctgatgacac ggggcgatct caaaagttat ctccggtctc tgaggccaga aatggagaat 3300
   aatccagtcc tagcacctcc aagcctgagc aagatgattc agatggccgg agagattgca 3360
   gacggcatgg catacctcaa cgccaataag ttcgtccaca gagaccttgc tgcccggaat 3420
   tgcatggtag ccgaagattt cacagtcaaa atcggagatt ttggtatgac gcgagatatc 3480
   tatgagacag actattaccg gaaaggaggg aaagggctgc tgcccgtgcg ctggatgtct 3540
   cctgagtccc tcaaggatgg agtcttcacc acttactcgg acgtctggtc cttcggggtc 3600
   gtcctctggg agatcgccac actggccgag cagccctacc agggcttgtc caacgagcaa 3660
   gtccttcgct tcgtcatgga gggcggcctt ctggacaagc cagacaactg tcctgacatg 3720
   ctgtttgaac tgatgcgcat gtgctggcag tataacccca agatgaggcc ttccttcctg 3780
   gagatcatca gcagcatcaa agaggagatg gagcctggct tccgggaggt ctccttctac 3840
   tacagcgagg agaacaagct gcccgagccg gaggagctgg acctggagcc agagaacatg 3900
   gagagcgtcc ccctggaccc ctcggcctcc tcgtcctccc tgccactgcc cgacagacac 3960
   tcaggacaca aggccgagaa cggccccggc cctggggtgc tggtcctccg cgccagcttc 4020
   gacgagagac agccttacgc ccacatgaac gggggccgca agaacgagcg ggccttgccg 4080
   ctgccccagt cttcgacctg cgaattcgtc accgacgcca aaaacataaa gaaaggcccg 4140
   gcgccattct atccgctgga agatggaacc gctggagagc aactgcataa ggctatgaag 4200
   agatacgccc tggttcctgg aacaattgct tttacagatg cacatatcga ggtggacatc 4260
   acttacgctg agtacttcga aatgtccgtt cggttggcag aagctatgaa acgatatggg 4320
   ctgaatacaa atcacagaat cgtcgtatgc agtgaaaact ctcttcaatt ctttatgccg 4380
   gtgttgggcg cgttatttat cggagttgca gttgcgcccg cgaacgacat ttataatgaa 4440
   cgtgaattgc tcaacagtat gggcatttcg cagcctaccg tggtgttcgt ttccaaaaag 4500
   gggttgcaaa aaattttgaa cgtgcaaaaa aagctcccaa tcatccaaaa aattattatc 4560
   atggattcta aaacggatta ccagggattt cagtcgatgt acacgttcgt cacatctcat 4620
   ctacctcccg gttttaatga atacgatttt gtgccagagt ccttcgatag ggacaagaca 4680
   attgcactga tcatgaactc ctctggatct actggtctgc ctaaaggtgt cgctctgcct 4740
   catagaactg cctgcgtgag attctcgcat gccagagatc ctatttttgg caatcaaatc 4800
   attccggata ctgcgatttt aagtgttgtt ccattccatc acggttttgg aatgtttact 4860
   acactcggat atttgatatg tggatttcga gtcgtcttaa tgtatagatt tgaagaagag 4920
   ctgtttctga ggagccttca ggattacaag attcaaagtg cgctgctggt gccaacccta 4980
   ttctccttct tcgccaaaag cactctgatt gacaaatacg atttatctaa tttacacgaa 5040
   attgcttctg gtggcgctcc cctctctaag gaagtcgggg aagcggttgc caagaggttc 5100
   catctgccag gtatcaggca aggatatggg ctcactgaga ctacatcagc tattctgatt 5160
   acacccgagg gggatgataa accgggcgcg gtcggtaaag ttgttccatt ttttgaagcg 5220
   aaggttgtgg atctggatac cgggaaaacg ctgggcgtta atcaaagagg cgaactgtgt 5280
   gtgagaggtc ctatgattat gtccggttat gtaaacaatc cggaagcgac caacgccttg 5340
   attgacaagg atggatggct acattctgga gacatagctt actgggacga agacgaacac 5400
   ttcttcatcg ttgaccgcct gaagtctctg attaagtaca aaggctatca ggtggctccc     5460
   gctgaattgg aatccatctt gctccaacac cccaacatct tcgacgcagg tgtcgcaggt     5520
   cttcccgacg atgacgccgg tgaacttccc gccgccgttg ttgttttgga gcacggaaag     5580
   acgatgacgg aaaaagagat cgtggattac gtcgccagtc aagtaacaac cgcgaaaaag     5640
   ttgcgcggag gagttgtgtt tgtggacgaa gtaccgaaag gtcttaccgg aaaactcgac     5700
   gcaagaaaaa tcagagagat cctcataaag gccaagaagg gcggaaagat cgccgtgtaa     5760
<210> 10
   <211> 1919
   <212> PRT
   <213> Artificial Sequence
<220>
   <223> hIGF1R-luc fusion protein
<400> 10
   Met Lys Ser Gly Ser Gly Gly Gly Ser Pro Thr Ser Leu Trp Gly Leu
   1               5                   10                  15
   Leu Phe Leu Ser Ala Ala Leu Ser Leu Trp Pro Thr Ser Gly Glu Ile
               20                  25                  30
   Cys Gly Pro Gly Ile Asp Ile Arg Asn Asp Tyr Gln Gln Leu Lys Arg
           35                  40                  45
   Leu Glu Asn Cys Thr Val Ile Glu Gly Tyr Leu His Ile Leu Leu Ile
       50                  55                  60
   Ser Lys Ala Glu Asp Tyr Arg Ser Tyr Arg Phe Pro Lys Leu Thr Val
   65                  70                  75                  80
   Ile Thr Glu Tyr Leu Leu Leu Phe Arg Val Ala Gly Leu Glu Ser Leu
                   85                  90                  95
   Gly Asp Leu Phe Pro Asn Leu Thr Val Ile Arg Gly Trp Lys Leu Phe
               100                 105                 110
   Tyr Asn Tyr Ala Leu Val Ile Phe Glu Met Thr Asn Leu Lys Asp Ile
           115                 120                 125
   Gly Leu Tyr Asn Leu Arg Asn Ile Thr Arg Gly Ala Ile Arg Ile Glu
       130                 135                 140
   Lys Asn Ala Asp Leu Cys Tyr Leu Ser Thr Val Asp Trp Ser Leu Ile
   145                 150                 155                 160
   Leu Asp Ala Val Ser Asn Asn Tyr Ile Val Gly Asn Lys Pro Pro Lys
                   165                 170                 175
   Glu Cys Gly Asp Leu Cys Pro Gly Thr Met Glu Glu Lys Pro Met Cys
               180                 185                 190
   Glu Lys Thr Thr Ile Asn Asn Glu Tyr Asn Tyr Arg Cys Trp Thr Thr
           195                 200                 205
   Asn Arg Cys Gln Lys Met Cys Pro Ser Thr Cys Gly Lys Arg Ala Cys
       210                 215                 220
   Thr Glu Asn Asn Glu Cys Cys His Pro Glu Cys Leu Gly Ser Cys Ser
   225                 230                 235                 240
   Ala Pro Asp Asn Asp Thr Ala Cys Val Ala Cys Arg His Tyr Tyr Tyr
                   245                 250                 255
   Ala Gly Val Cys Val Pro Ala Cys Pro Pro Asn Thr Tyr Arg Phe Glu
               260                 265                 270
   Gly Trp Arg Cys Val Asp Arg Asp Phe Cys Ala Asn Ile Leu Ser Ala
           275                 280                 285
   Glu Ser Ser Asp Ser Glu Gly Phe Val Ile His Asp Gly Glu Cys Met
       290                 295                 300
   Gln Glu Cys Pro Ser Gly Phe Ile Arg Asn Gly Ser Gln Ser Met Tyr
   305                 310                 315                 320
   Cys Ile Pro Cys Glu Gly Pro Cys Pro Lys Val Cys Glu Glu Glu Lys
                   325                 330                 335
   Lys Thr Lys Thr Ile Asp Ser Val Thr Ser Ala Gln Met Leu Gln Gly
               340                 345                 350
   Cys Thr Ile Phe Lys Gly Asn Leu Leu Ile Asn Ile Arg Arg Gly Asn
           355                 360                 365
   Asn Ile Ala Ser Glu Leu Glu Asn Phe Met Gly Leu Ile Glu Val Val
       370                 375                 380
   Thr Gly Tyr Val Lys Ile Arg His Ser His Ala Leu Val Ser Leu Ser
   385                 390                 395                 400
   Phe Leu Lys Asn Leu Arg Leu Ile Leu Gly Glu Glu Gln Leu Glu Gly
                   405                 410                 415
   Asn Tyr Ser Phe Tyr Val Leu Asp Asn Gln Asn Leu Gln Gln Leu Trp
               420                 425                 430
   Asp Trp Asp His Arg Asn Leu Thr Ile Lys Ala Gly Lys Met Tyr Phe
           435                 440                 445
   Ala Phe Asn Pro Lys Leu Cys Val Ser Glu Ile Tyr Arg Met Glu Glu
       450                 455                 460
   Val Thr Gly Thr Lys Gly Arg Gln Ser Lys Gly Asp Ile Asn Thr Arg
   465                 470                 475                 480
   Asn Asn Gly Glu Arg Ala Ser Cys Glu Ser Asp Val Leu His Phe Thr
                   485                 490                 495
   Ser Thr Thr Thr Ser Lys Asn Arg Ile Ile Ile Thr Trp His Arg Tyr
               500                 505                 510
   Arg Pro Pro Asp Tyr Arg Asp Leu Ile Ser Phe Thr Val Tyr Tyr Lys
           515                 520                 525
   Glu Ala Pro Phe Lys Asn Val Thr Glu Tyr Asp Gly Gln Asp Ala Cys
       530                 535                 540
   Gly Ser Asn Ser Trp Asn Met Val Asp Val Asp Leu Pro Pro Asn Lys
   545                 550                 555                 560
   Asp Val Glu Pro Gly Ile Leu Leu His Gly Leu Lys Pro Trp Thr Gln
                   565                 570                 575
   Tyr Ala Val Tyr Val Lys Ala Val Thr Leu Thr Met Val Glu Asn Asp
               580                 585                 590
   His Ile Arg Gly Ala Lys Ser Glu Ile Leu Tyr Ile Arg Thr Asn Ala
           595                 600                 605
   Ser Val Pro Ser Ile Pro Leu Asp Val Leu Ser Ala Ser Asn Ser Ser
       610                 615                 620
   Ser Gln Leu Ile Val Lys Trp Asn Pro Pro Ser Leu Pro Asn Gly Asn
   625                 630                 635                 640
   Leu Ser Tyr Tyr Ile Val Arg Trp Gln Arg Gln Pro Gln Asp Gly Tyr
                   645                 650                 655
   Leu Tyr Arg His Asn Tyr Cys Ser Lys Asp Lys Ile Pro Ile Arg Lys
               660                 665                 670
   Tyr Ala Asp Gly Thr Ile Asp Ile Glu Glu Val Thr Glu Asn Pro Lys
           675                 680                 685
   Thr Glu Val Cys Gly Gly Glu Lys Gly Pro Cys Cys Ala Cys Pro Lys
       690                 695                 700
   Thr Glu Ala Glu Lys Gln Ala Glu Lys Glu Glu Ala Glu Tyr Arg Lys
   705                 710                 715                 720
   Val Phe Glu Asn Phe Leu His Asn Ser Ile Phe Val Pro Arg Pro Glu
                   725                 730                 735
   Arg Lys Arg Arg Asp Val Met Gln Val Ala Asn Thr Thr Met Ser Ser
               740                 745                 750
   Arg Ser Arg Asn Thr Thr Ala Ala Asp Thr Tyr Asn Ile Thr Asp Pro
           755                 760                 765
   Glu Glu Leu Glu Thr Glu Tyr Pro Phe Phe Glu Ser Arg Val Asp Asn
       770                 775                 780
   Lys Glu Arg Thr Val Ile Ser Asn Leu Arg Pro Phe Thr Leu Tyr Arg
   785                 790                 795                 800
   Ile Asp Ile His Ser Cys Asn His Glu Ala Glu Lys Leu Gly Cys Ser
                   805                 810                 815
   Ala Ser Asn Phe Val Phe Ala Arg Thr Met Pro Ala Glu Gly Ala Asp
               820                 825                 830
   Asp Ile Pro Gly Pro Val Thr Trp Glu Pro Arg Pro Glu Asn Ser Ile
           835                 840                 845
   Phe Leu Lys Trp Pro Glu Pro Glu Asn Pro Asn Gly Leu Ile Leu Met
       850                 855                 860
   Tyr Glu Ile Lys Tyr Gly Ser Gln Val Glu Asp Gln Arg Glu Cys Val
   865                 870                 875                 880
   Ser Arg Gln Glu Tyr Arg Lys Tyr Gly Gly Ala Lys Leu Asn Arg Leu
                   885                 890                 895
   Asn Pro Gly Asn Tyr Thr Ala Arg Ile Gln Ala Thr Ser Leu Ser Gly
               900                 905                 910
   Asn Gly Ser Trp Thr Asp Pro Val Phe Phe Tyr Val Gln Ala Lys Thr
           915                 920                 925
   Gly Tyr Glu Asn Phe Ile His Leu Ile Ile Ala Leu Pro Val Ala Val
       930                 935                 940
   Leu Leu Ile Val Gly Gly Leu Val Ile Met Leu Tyr Val Phe His Arg
   945                 950                 955                 960
   Lys Arg Asn Asn Ser Arg Leu Gly Asn Gly Val Leu Tyr Ala Ser Val
                   965                 970                 975
   Asn Pro Glu Tyr Phe Ser Ala Ala Asp Val Tyr Val Pro Asp Glu Trp
               980                 985                 990
   Glu Val Ala Arg Glu Lys Ile Thr Met Ser Arg Glu Leu Gly Gln Gly
           995                 1000                1005
   Ser Phe Gly Met Val Tyr Glu Gly Val Ala Lys Gly Val Val Lys Asp
       1010                1015                1020
   Glu Pro Glu Thr Arg Val Ala Ile Lys Thr Val Asn Glu Ala Ala Ser
   1025                1030                1035                1040
   Met Arg Glu Arg Ile Glu Phe Leu Asn Glu Ala Ser Val Met Lys Glu
                   1045                1050                1055
   Phe Asn Cys His His Val Val Arg Leu Leu Gly Val Val Ser Gln Gly
               1060                1065                1070
   Gln Pro Thr Leu Val Ile Met Glu Leu Met Thr Arg Gly Asp Leu Lys
           1075                1080                1085
   Ser Tyr Leu Arg Ser Leu Arg Pro Glu Met Glu Asn Asn Pro Val Leu
       1090                1095                1100
   Ala Pro Pro Ser Leu Ser Lys Met Ile Gln Met Ala Gly Glu Ile Ala
   1105                1110                1115                1120
   Asp Gly Met Ala Tyr Leu Asn Ala Asn Lys Phe Val His Arg Asp Leu
                   1125                1130                1135
   Ala Ala Arg Asn Cys Met Val Ala Glu Asp Phe Thr Val Lys Ile Gly
               1140                1145                1150
   Asp Phe Gly Met Thr Arg Asp Ile Tyr Glu Thr Asp Tyr Tyr Arg Lys
           1155                1160                1165
   Gly Gly Lys Gly Leu Leu Pro Val Arg Trp Met Ser Pro Glu Ser Leu
       1170                1175                1180
   Lys Asp Gly Val Phe Thr Thr Tyr Ser Asp Val Trp Ser Phe Gly Val
   1185                1190                1195                1200
   Val Leu Trp Glu Ile Ala Thr Leu Ala Glu Gln Pro Tyr Gln Gly Leu
                   1205                1210                1215
   Ser Asn Glu Gln Val Leu Arg Phe Val Met Glu Gly Gly Leu Leu Asp
               1220                1225                1230
   Lys Pro Asp Asn Cys Pro Asp Met Leu Phe Glu Leu Met Arg Met Cys
           1235                1240                1245
   Trp Gln Tyr Asn Pro Lys Met Arg Pro Ser Phe Leu Glu Ile Ile Ser
       1250                1255                1260
   Ser Ile Lys Glu Glu Met Glu Pro Gly Phe Arg Glu Val Ser Phe Tyr
   1265                1270                1275                1280
   Tyr Ser Glu Glu Asn Lys Leu Pro Glu Pro Glu Glu Leu Asp Leu Glu
                   1285                1290                1295
   Pro Glu Asn Met Glu Ser Val Pro Leu Asp Pro Ser Ala Ser Ser Ser
               1300                1305                1310
   Ser Leu Pro Leu Pro Asp Arg His Ser Gly His Lys Ala Glu Asn Gly
           1315                1320                1325
   Pro Gly Pro Gly Val Leu Val Leu Arg Ala Ser Phe Asp Glu Arg Gln
       1330                1335                1340
   Pro Tyr Ala His Met Asn Gly Gly Arg Lys Asn Glu Arg Ala Leu Pro
   1345                1350                1355                1360
   Leu Pro Gln Ser Ser Thr Cys Glu Phe Val Thr Asp Ala Lys Asn Ile
                   1365                1370                1375
   Lys Lys Gly Pro Ala Pro Phe Tyr Pro Leu Glu Asp Gly Thr Ala Gly
               1380                1385                1390
   Glu Gln Leu His Lys Ala Met Lys Arg Tyr Ala Leu Val Pro Gly Thr
           1395                1400                1405
   Ile Ala Phe Thr Asp Ala His Ile Glu Val Asp Ile Thr Tyr Ala Glu
       1410                1415                1420
   Tyr Phe Glu Met Ser Val Arg Leu Ala Glu Ala Met Lys Arg Tyr Gly
   1425                1430                1435                1440
   Leu Asn Thr Asn His Arg Ile Val Val Cys Ser Glu Asn Ser Leu Gln
                   1445                1450                1455
   Phe Phe Met Pro Val Leu Gly Ala Leu Phe Ile Gly Val Ala Val Ala
               1460                1465                1470
   Pro Ala Asn Asp Ile Tyr Asn Glu Arg Glu Leu Leu Asn Ser Met Gly
           1475                1480                1485
   Ile Ser Gln Pro Thr Val Val Phe Val Ser Lys Lys Gly Leu Gln Lys
       1490                1495                1500
   Ile Leu Asn Val Gln Lys Lys Leu Pro Ile Ile Gln Lys Ile Ile Ile
   1505                1510                1515                1520
   Met Asp Ser Lys Thr Asp Tyr Gln Gly Phe Gln Ser Met Tyr Thr Phe
                   1525                1530                1535
   Val Thr Ser His Leu Pro Pro Gly Phe Asn Glu Tyr Asp Phe Val Pro
               1540                1545                1550
   Glu Ser Phe Asp Arg Asp Lys Thr Ile Ala Leu Ile Met Asn Ser Ser
           1555                1560                1565
   Gly Ser Thr Gly Leu Pro Lys Gly Val Ala Leu Pro His Arg Thr Ala
       1570                1575                1580
   Cys Val Arg Phe Ser His Ala Arg Asp Pro Ile Phe Gly Asn Gln Ile
   1585                1590                1595                1600
   Ile Pro Asp Thr Ala Ile Leu Ser Val Val Pro Phe His His Gly Phe
                   1605                1610                1615
   Gly Met Phe Thr Thr Leu Gly Tyr Leu Ile Cys Gly Phe Arg Val Val
               1620                1625                1630
   Leu Met Tyr Arg Phe Glu Glu Glu Leu Phe Leu Arg Ser Leu Gln Asp
           1635                1640                1645
   Tyr Lys Ile Gln Ser Ala Leu Leu Val Pro Thr Leu Phe Ser Phe Phe
       1650                1655                1660
   Ala Lys Ser Thr Leu Ile Asp Lys Tyr Asp Leu Ser Asn Leu His Glu
   1665                1670                1675                1680
   Ile Ala Ser Gly Gly Ala Pro Leu Ser Lys Glu Val Gly Glu Ala Val
                   1685                1690                1695
   Ala Lys Arg Phe His Leu Pro Gly Ile Arg Gln Gly Tyr Gly Leu Thr
               1700                1705                1710
   Glu Thr Thr Ser Ala Ile Leu Ile Thr Pro Glu Gly Asp Asp Lys Pro
           1715                1720                1725
   Gly Ala Val Gly Lys Val Val Pro Phe Phe Glu Ala Lys Val Val Asp
       1730                1735                1740
   Leu Asp Thr Gly Lys Thr Leu Gly Val Asn Gln Arg Gly Glu Leu Cys
   1745                1750                1755                1760
   Val Arg Gly Pro Met Ile Met Ser Gly Tyr Val Asn Asn Pro Glu Ala
                   1765                1770                1775
   Thr Asn Ala Leu Ile Asp Lys Asp Gly Trp Leu His Ser Gly Asp Ile
               1780                1785                1790
   Ala Tyr Trp Asp Glu Asp Glu His Phe Phe Ile Val Asp Arg Leu Lys
           1795                1800                1805
   Ser Leu Ile Lys Tyr Lys Gly Tyr Gln Val Ala Pro Ala Glu Leu Glu
       1810                1815                1820
   Ser Ile Leu Leu Gln His Pro Asn Ile Phe Asp Ala Gly Val Ala Gly
   1825                1830                1835                1840
   Leu Pro Asp Asp Asp Ala Gly Glu Leu Pro Ala Ala Val Val Val Leu
                   1845                1850                1855
   Glu His Gly Lys Thr Met Thr Glu Lys Glu Ile Val Asp Tyr Val Ala
               1860                1865                1870
   Ser Gln Val Thr Thr Ala Lys Lys Leu Arg Gly Gly Val Val Phe Val
           1875                1880                1885
   Asp Glu Val Pro Lys Gly Leu Thr Gly Lys Leu Asp Ala Arg Lys Ile
       1890                1895                1900
   Arg Glu Ile Leu Ile Lys Ala Lys Lys Gly Gly Lys Ile Ala Val
   1905                1910                1915
<210> 11
   <211> 4113
   <212> DNA
   <213> Homo sapiens
<220>
   <221> source
   <222> 1..4113
   <223> /organism="Homo sapiens" /note="cDNA insulin receptor" /mol_type="unassigned DNA"
<400> 11
   atgggcaccg ggggccggcg gggggcggcg gccgcgccgc tgctggtggc ggtggccgcg      60
   ctgctactgg gcgccgcggg ccacctgtac cccggagagg tgtgtcccgg catggatatc     120
   cggaacaacc tcactaggtt gcatgagctg gagaattgct ctgtcatcga aggacacttg     180
   cagatactct tgatgttcaa aacgaggccc gaagatttcc gagacctcag tttccccaaa     240
   ctcatcatga tcactgatta cttgctgctc ttccgggtct atgggctcga gagcctgaag     300
   gacctgttcc ccaacctcac ggtcatccgg ggatcacgac tgttctttaa ctacgcgctg     360
   gtcatcttcg agatggttca cctcaaggaa ctcggcctct acaacctgat gaacatcacc     420
   cggggttctg tccgcatcga gaagaacaat gagctctgtt acttggccac tatcgactgg     480
   tcccgtatcc tggattccgt ggaggataat tacatcgtgt tgaacaaaga tgacaacgag     540
   gagtgtggag acatctgtcc gggtaccgcg aagggcaaga ccaactgccc cgccaccgtc     600
   atcaacgggc agtttgtcga acgatgttgg actcatagtc actgccagaa agtttgcccg     660
   accatctgta agtcacacgg ctgcaccgcc gaaggcctct gttgccacag cgagtgcctg     720
   ggcaactgtt ctcagcccga cgaccccacc aagtgcgtgg cctgccgcaa cttctacctg     780
   gacggcaggt gtgtggagac ctgcccgccc ccgtactacc acttccagga ctggcgctgt     840
   gtgaacttca gcttctgcca ggacctgcac cacaaatgca agaactcgcg gaggcagggc     900
   tgccaccagt acgtcattca caacaacaag tgcatccctg agtgtccctc cgggtacacg     960
   atgaattcca gcaacttgct gtgcacccca tgcctgggtc cctgtcccaa ggtgtgccac    1020
   ctcctagaag gcgagaagac catcgactcg gtgacgtctg cccaggagct ccgaggatgc    1080
   accgtcatca acgggagtct gatcatcaac attcgaggag gcaacaatct ggcagctgag    1140
   ctagaagcca acctcggcct cattgaagaa atttcagggt atctaaaaat ccgccgatcc    1200
   tacgctctgg tgtcactttc cttcttccgg aagttacgtc tgattcgagg agagaccttg 1260
   gaaattggga actactcctt ctatgccttg gacaaccaga acctaaggca gctctgggac 1320
   tggagcaaac acaacctcac catcactcag gggaaactct tcttccacta taaccccaaa 1380
   ctctgcttgt cagaaatcca caagatggaa gaagtttcag gaaccaaggg gcgccaggag 1440
   agaaacgaca ttgccctgaa gaccaatggg gaccaggcat cctgtgaaaa tgagttactt 1500
   aaattttctt acattcggac atcttttgac aagatcttgc tgagatggga gccgtactgg 1560
   ccccccgact tccgagacct cttggggttc atgctgttct acaaagaggc cccttatcag 1620
   aatgtgacgg agttcgacgg gcaggatgcg tgtggttcca acagttggac ggtggtagac 1680
   attgacccac ccctgaggtc caacgacccc aaatcacaga accacccagg gtggctgatg 1740
   cggggtctca agccctggac ccagtatgcc atctttgtga agaccctggt caccttttcg 1800
   gatgaacgcc ggacctatgg ggccaagagt gacatcattt atgtccagac agatgccacc 1860
   aacccctctg tgcccctgga tccaatctca gtgtctaact catcatccca gattattctg 1920
   aagtggaaac caccctccga ccccaatggc aacatcaccc actacctggt tttctgggag 1980
   aggcaggcgg aagacagtga gctgttcgag ctggattatt gcctcaaagg gctgaagctg 2040
   ccctcgagga cctggtctcc accattcgag tctgaagatt ctcagaagca caaccagagt 2100
   gagtatgagg attcggccgg cgaatgctgc tcctgtccaa agacagactc tcagatcctg 2160
   aaggagctgg aggagtcctc gtttaggaag acgtttgagg attacctgca caacgtggtt 2220
   ttcgtcccca ggccatctcg gaaacgcagg tcccttggcg atgttgggaa tgtgacggtg 2280
   gccgtgccca cggtggcagc tttccccaac acttcctcga ccagcgtgcc cacgagtccg 2340
   gaggagcaca ggccttttga gaaggtggtg aacaaggagt cgctggtcat ctccggcttg 2400
   cgacacttca cgggctatcg catcgagctg caggcttgca accaggacac ccctgaggaa 2460
   cggtgcagtg tggcagccta cgtcagtgcg aggaccatgc ctgaagccaa ggctgatgac 2520
   attgttggcc ctgtgacgca tgaaatcttt gagaacaacg tcgtccactt gatgtggcag 2580
   gagccgaagg agcccaatgg tctgatcgtg ctgtatgaag tgagttatcg gcgatatggt 2640
   gatgaggagc tgcatctctg cgtctcccgc aagcacttcg ctctggaacg gggctgcagg 2700
   ctgcgtgggc tgtcaccggg gaactacagc gtgcgaatcc gggccacctc ccttgcgggc 2760
   aacggctctt ggacggaacc cacctatttc tacgtgacag actatttaga cgtcccgtca 2820
   aatattgcaa aaattatcat cggccccctc atctttgtct ttctcttcag tgttgtgatt 2880
   ggaagtattt atctattcct gagaaagagg cagccagatg ggccgctggg accgctttac 2940
   gcttcttcaa accctgagta tctcagtgcc agtgatgtgt ttccatgctc tgtgtacgtg 3000
   ccggacgagt gggaggtgtc tcgagagaag atcaccctcc ttcgagagct ggggcagggc 3060
   tccttcggca tggtgtatga gggcaatgcc agggacatca tcaagggtga ggcagagacc 3120
   cgcgtggcgg tgaagacggt caacgagtca gccagtctcc gagagcggat tgagttcctc 3180
   aatgaggcct cggtcatgaa gggcttcacc tgccatcatg tggtgcgcct cctgggagtg 3240
   gtgtccaagg gccagcccac gctggtggtg atggagctga tggctcacgg agacctgaag 3300
   agctacctcc gttctctgcg gccagaggct gagaataatc ctggccgccc tccccctacc 3360
   cttcaagaga tgattcagat ggcggcagag attgctgacg ggatggccta cctgaacgcc 3420
   aagaagtttg tgcatcggga cctggcagcg agaaactgca tggtcgccca tgattttact 3480
   gtcaaaattg gagactttgg aatgaccaga gacatctatg aaacggatta ctaccggaaa 3540
   gggggcaagg gtctgctccc tgtacggtgg atggcaccgg agtccctgaa ggatggggtc 3600
   ttcaccactt cttctgacat gtggtccttt ggcgtggtcc tttgggaaat caccagcttg 3660
   gcagaacagc cttaccaagg cctgtctaat gaacaggtgt tgaaatttgt catggatgga 3720
   gggtatctgg atcaacccga caactgtcca gagagagtca ctgacctcat gcgcatgtgc 3780
   tggcaattca accccaagat gaggccaacc ttcctggaga ttgtcaacct gctcaaggac 3840
   gacctgcacc ccagctttcc agaggtgtcg ttcttccaca gcgaggagaa caaggctccc 3900
   gagagtgagg agctggagat ggagtttgag gacatggaga atgtgcccct ggaccgttcc 3960
   tcgcactgtc agagggagga ggcggggggc cgggatggag ggtcctcgct gggtttcaag 4020
   cggagctacg aggaacacat cccttacaca cacatgaacg gaggcaagaa aaacgggcgg 4080
   attctgacct tgcctcggtc caatccttcc taa 4113
<210> 12
   <211> 1370
   <212> PRT
   <213> Homo sapiens
<220>
   <223> insulin receptor
<400> 12
<210> 13
   <211> 5769
   <212> DNA
   <213> Artificial Sequence
<220>
   <221> source
   <222> 1..5769
   <223> /organism="Artificial Sequence" /note="human insulin receptor-luc fusion" /mol_type="unassigned DNA"
<400> 13
   atgggcaccg ggggccggcg gggggcggcg gccgcgccgc tgctggtggc ggtggccgcg        60
   ctgctactgg gcgccgcggg ccacctgtac cccggagagg tgtgtcccgg catggatatc       120
   cggaacaacc tcactaggtt gcatgagctg gagaattgct ctgtcatcga aggacacttg       180
   cagatactct tgatgttcaa aacgaggccc gaagatttcc gagacctcag tttccccaaa       240
   ctcatcatga tcactgatta cttgctgctc ttccgggtct atgggctcga gagcctgaag       300
   gacctgttcc ccaacctcac ggtcatccgg ggatcacgac tgttctttaa ctacgcgctg       360
   gtcatcttcg agatggttca cctcaaggaa ctcggcctct acaacctgat gaacatcacc       420
   cggggttctg tccgcatcga gaagaacaat gagctctgtt acttggccac tatcgactgg       480
   tcccgtatcc tggattccgt ggaggataat tacatcgtgt tgaacaaaga tgacaacgag       540
   gagtgtggag acatctgtcc gggtaccgcg aagggcaaga ccaactgccc cgccaccgtc       600
   atcaacgggc agtttgtcga acgatgttgg actcatagtc actgccagaa agtttgcccg    660
   accatctgta agtcacacgg ctgcaccgcc gaaggcctct gttgccacag cgagtgcctg    720
   ggcaactgtt ctcagcccga cgaccccacc aagtgcgtgg cctgccgcaa cttctacctg    780
   gacggcaggt gtgtggagac ctgcccgccc ccgtactacc acttccagga ctggcgctgt    840
   gtgaacttca gcttctgcca ggacctgcac cacaaatgca agaactcgcg gaggcagggc    900
   tgccaccagt acgtcattca caacaacaag tgcatccctg agtgtccctc cgggtacacg    960
   atgaattcca gcaacttgct gtgcacccca tgcctgggtc cctgtcccaa ggtgtgccac   1020
   ctcctagaag gcgagaagac catcgactcg gtgacgtctg cccaggagct ccgaggatgc   1080
   accgtcatca acgggagtct gatcatcaac attcgaggag gcaacaatct ggcagctgag   1140
   ctagaagcca acctcggcct cattgaagaa atttcagggt atctaaaaat ccgccgatcc   1200
   tacgctctgg tgtcactttc cttcttccgg aagttacgtc tgattcgagg agagaccttg   1260
   gaaattggga actactcctt ctatgccttg gacaaccaga acctaaggca gctctgggac   1320
   tggagcaaac acaacctcac catcactcag gggaaactct tcttccacta taaccccaaa   1380
   ctctgcttgt cagaaatcca caagatggaa gaagtttcag gaaccaaggg gcgccaggag   1440
   agaaacgaca ttgccctgaa gaccaatggg gaccaggcat cctgtgaaaa tgagttactt   1500
   aaattttctt acattcggac atcttttgac aagatcttgc tgagatggga gccgtactgg   1560
   ccccccgact tccgagacct cttggggttc atgctgttct acaaagaggc cccttatcag   1620
   aatgtgacgg agttcgacgg gcaggatgcg tgtggttcca acagttggac ggtggtagac   1680
   attgacccac ccctgaggtc caacgacccc aaatcacaga accacccagg gtggctgatg   1740
   cggggtctca agccctggac ccagtatgcc atctttgtga agaccctggt caccttttcg   1800
   gatgaacgcc ggacctatgg ggccaagagt gacatcattt atgtccagac agatgccacc   1860
   aacccctctg tgcccctgga tccaatctca gtgtctaact catcatccca gattattctg   1920
   aagtggaaac caccctccga ccccaatggc aacatcaccc actacctggt tttctgggag   1980
   aggcaggcgg aagacagtga gctgttcgag ctggattatt gcctcaaagg gctgaagctg   2040
   ccctcgagga cctggtctcc accattcgag tctgaagatt ctcagaagca caaccagagt   2100
   gagtatgagg attcggccgg cgaatgctgc tcctgtccaa agacagactc tcagatcctg   2160
   aaggagctgg aggagtcctc gtttaggaag acgtttgagg attacctgca caacgtggtt   2220
   ttcgtcccca ggccatctcg gaaacgcagg tcccttggcg atgttgggaa tgtgacggtg   2280
   gccgtgccca cggtggcagc tttccccaac acttcctcga ccagcgtgcc cacgagtccg   2340
   gaggagcaca ggccttttga gaaggtggtg aacaaggagt cgctggtcat ctccggcttg   2400
   cgacacttca cgggctatcg catcgagctg caggcttgca accaggacac ccctgaggaa   2460
   cggtgcagtg tggcagccta cgtcagtgcg aggaccatgc ctgaagccaa ggctgatgac   2520
   attgttggcc ctgtgacgca tgaaatcttt gagaacaacg tcgtccactt gatgtggcag 2580
   gagccgaagg agcccaatgg tctgatcgtg ctgtatgaag tgagttatcg gcgatatggt 2640
   gatgaggagc tgcatctctg cgtctcccgc aagcacttcg ctctggaacg gggctgcagg 2700
   ctgcgtgggc tgtcaccggg gaactacagc gtgcgaatcc gggccacctc ccttgcgggc 2760
   aacggctctt ggacggaacc cacctatttc tacgtgacag actatttaga cgtcccgtca 2820
   aatattgcaa aaattatcat cggccccctc atctttgtct ttctcttcag tgttgtgatt 2880
   ggaagtattt atctattcct gagaaagagg cagccagatg ggccgctggg accgctttac 2940
   gcttcttcaa accctgagta tctcagtgcc agtgatgtgt ttccatgctc tgtgtacgtg 3000
   ccggacgagt gggaggtgtc tcgagagaag atcaccctcc ttcgagagct ggggcagggc 3060
   tccttcggca tggtgtatga gggcaatgcc agggacatca tcaagggtga ggcagagacc 3120
   cgcgtggcgg tgaagacggt caacgagtca gccagtctcc gagagcggat tgagttcctc 3180
   aatgaggcct cggtcatgaa gggcttcacc tgccatcatg tggtgcgcct cctgggagtg 3240
   gtgtccaagg gccagcccac gctggtggtg atggagctga tggctcacgg agacctgaag 3300
   agctacctcc gttctctgcg gccagaggct gagaataatc ctggccgccc tccccctacc 3360
   cttcaagaga tgattcagat ggcggcagag attgctgacg ggatggccta cctgaacgcc 3420
   aagaagtttg tgcatcggga cctggcagcg agaaactgca tggtcgccca tgattttact 3480
   gtcaaaattg gagactttgg aatgaccaga gacatctatg aaacggatta ctaccggaaa 3540
   gggggcaagg gtctgctccc tgtacggtgg atggcaccgg agtccctgaa ggatggggtc 3600
   ttcaccactt cttctgacat gtggtccttt ggcgtggtcc tttgggaaat caccagcttg 3660
   gcagaacagc cttaccaagg cctgtctaat gaacaggtgt tgaaatttgt catggatgga 3720
   gggtatctgg atcaacccga caactgtcca gagagagtca ctgacctcat gcgcatgtgc 3780
   tggcaattca accccaagat gaggccaacc ttcctggaga ttgtcaacct gctcaaggac 3840
   gacctgcacc ccagctttcc agaggtgtcg ttcttccaca gcgaggagaa caaggctccc 3900
   gagagtgagg agctggagat ggagtttgag gacatggaga atgtgcccct ggaccgttcc 3960
   tcgcactgtc agagggagga ggcggggggc cgggatggag ggtcctcgct gggtttcaag 4020
   cggagctacg aggaacacat cccttacaca cacatgaacg gaggcaagaa aaacgggcgg 4080
   attctgacct tgcctcggtc caatccttcc caattggtca ccgacgccaa aaacataaag 4140
   aaaggcccgg cgccattcta tccgctggaa gatggaaccg ctggagagca actgcataag 4200
   gctatgaaga gatacgccct ggttcctgga acaattgctt ttacagatgc acatatcgag 4260
   gtggacatca cttacgctga gtacttcgaa atgtccgttc ggttggcaga agctatgaaa 4320
   cgatatgggc tgaatacaaa tcacagaatc gtcgtatgca gtgaaaactc tcttcaattc 4380
   tttatgccgg tgttgggcgc gttatttatc ggagttgcag ttgcgcccgc gaacgacatt 4440
   tataatgaac gtgaattgct caacagtatg ggcatttcgc agcctaccgt ggtgttcgtt 4500
   tccaaaaagg ggttgcaaaa aattttgaac gtgcaaaaaa agctcccaat catccaaaaa 4560
   attattatca tggattctaa aacggattac cagggatttc agtcgatgta cacgttcgtc 4620
   acatctcatc tacctcccgg ttttaatgaa tacgattttg tgccagagtc cttcgatagg 4680
   gacaagacaa ttgcactgat catgaactcc tctggatcta ctggtctgcc taaaggtgtc 4740
   gctctgcctc atagaactgc ctgcgtgaga ttctcgcatg ccagagatcc tatttttggc 4800
   aatcaaatca ttccggatac tgcgatttta agtgttgttc cattccatca cggttttgga 4860
   atgtttacta cactcggata tttgatatgt ggatttcgag tcgtcttaat gtatagattt 4920
   gaagaagagc tgtttctgag gagccttcag gattacaaga ttcaaagtgc gctgctggtg 4980
   ccaaccctat tctccttctt cgccaaaagc actctgattg acaaatacga tttatctaat 5040
   ttacacgaaa ttgcttctgg tggcgctccc ctctctaagg aagtcgggga agcggttgcc 5100
   aagaggttcc atctgccagg tatcaggcaa ggatatgggc tcactgagac tacatcagct 5160
   attctgatta cacccgaggg ggatgataaa ccgggcgcgg tcggtaaagt tgttccattt 5220
   tttgaagcga aggttgtgga tctggatacc gggaaaacgc tgggcgttaa tcaaagaggc 5280
   gaactgtgtg tgagaggtcc tatgattatg tccggttatg taaacaatcc ggaagcgacc 5340
   aacgccttga ttgacaagga tggatggcta cattctggag acatagctta ctgggacgaa 5400
   gacgaacact tcttcatcgt tgaccgcctg aagtctctga ttaagtacaa aggctatcag 5460
   gtggctcccg ctgaattgga atccatcttg ctccaacacc ccaacatctt cgacgcaggt 5520
   gtcgcaggtc ttcccgacga tgacgccggt gaacttcccg ccgccgttgt tgttttggag 5580
   cacggaaaga cgatgacgga aaaagagatc gtggattacg tcgccagtca agtaacaacc 5640
   gcgaaaaagt tgcgcggagg agttgtgttt gtggacgaag taccgaaagg tcttaccgga 5700
   aaactcgacg caagaaaaat cagagagatc ctcataaagg ccaagaaggg cggaaagatc 5760
   gccgtgtaa 5769
<210> 14
   <211> 1922
   <212> PRT
   <213> Artificial Sequence
<220>
   <223> hIR-luc fusion protein
<400> 14
   Met Gly Thr Gly Gly Arg Arg Gly Ala Ala Ala Ala Pro Leu Leu Val
   1               5                   10                  15
   Ala Val Ala Ala Leu Leu Leu Gly Ala Ala Gly His Leu Tyr Pro Gly
               20                  25                  30
   Glu Val Cys Pro Gly Met Asp Ile Arg Asn Asn Leu Thr Arg Leu His
           35                  40                  45
   Glu Leu Glu Asn Cys Ser Val Ile Glu Gly His Leu Gln Ile Leu Leu
       50                  55                  60
   Met Phe Lys Thr Arg Pro Glu Asp Phe Arg Asp Leu Ser Phe Pro Lys
   65                  70                  75                  80
   Leu Ile Met Ile Thr Asp Tyr Leu Leu Leu Phe Arg Val Tyr Gly Leu
                   85                  90                  95
   Glu Ser Leu Lys Asp Leu Phe Pro Asn Leu Thr Val Ile Arg Gly Ser
               100                 105                 110
   Arg Leu Phe Phe Asn Tyr Ala Leu Val Ile Phe Glu Met Val His Leu
           115                 120                 125
   Lys Glu Leu Gly Leu Tyr Asn Leu Met Asn Ile Thr Arg Gly Ser Val
       130                 135                 140
   Arg Ile Glu Lys Asn Asn Glu Leu Cys Tyr Leu Ala Thr Ile Asp Trp
   145                 150                 155                 160
   Ser Arg Ile Leu Asp Ser Val Glu Asp Asn Tyr Ile Val Leu Asn Lys
                   165                 170                 175
   Asp Asp Asn Glu Glu Cys Gly Asp Ile Cys Pro Gly Thr Ala Lys Gly
               180                 185                 190
   Lys Thr Asn Cys Pro Ala Thr Val Ile Asn Gly Gln Phe Val Glu Arg
           195                 200                 205
   Cys Trp Thr His Ser His Cys Gln Lys Val Cys Pro Thr Ile Cys Lys
       210                 215                 220
   Ser His Gly Cys Thr Ala Glu Gly Leu Cys Cys His Ser Glu Cys Leu
   225                 230                 235                 240
   Gly Asn Cys Ser Gln Pro Asp Asp Pro Thr Lys Cys Val Ala Cys Arg
                   245                 250                 255
   Asn Phe Tyr Leu Asp Gly Arg Cys Val Glu Thr Cys Pro Pro Pro Tyr
               260                 265                 270
   Tyr His Phe Gln Asp Trp Arg Cys Val Asn Phe Ser Phe Cys Gln Asp
           275                 280                 285
   Leu His His Lys Cys Lys Asn Ser Arg Arg Gln Gly Cys His Gln Tyr
       290                 295                 300
   Val Ile His Asn Asn Lys Cys Ile Pro Glu Cys Pro Ser Gly Tyr Thr
   305                 310                 315                 320
   Met Asn Ser Ser Asn Leu Leu Cys Thr Pro Cys Leu Gly Pro Cys Pro
                   325                 330                 335
   Lys Val Cys His Leu Leu Glu Gly Glu Lys Thr Ile Asp Ser Val Thr
               340                 345                 350
   Ser Ala Gln Glu Leu Arg Gly Cys Thr Val Ile Asn Gly Ser Leu Ile
           355                 360                 365
   Ile Asn Ile Arg Gly Gly Asn Asn Leu Ala Ala Glu Leu Glu Ala Asn
       370                 375                 380
   Leu Gly Leu Ile Glu Glu Ile Ser Gly Tyr Leu Lys Ile Arg Arg Ser
   385                 390                 395                 400
   Tyr Ala Leu Val Ser Leu Ser Phe Phe Arg Lys Leu Arg Leu Ile Arg
                   405                 410                 415
   Gly Glu Thr Leu Glu Ile Gly Asn Tyr Ser Phe Tyr Ala Leu Asp Asn
               420                 425                 430
   Gln Asn Leu Arg Gln Leu Trp Asp Trp Ser Lys His Asn Leu Thr Ile
           435                 440                 445
   Thr Gln Gly Lys Leu Phe Phe His Tyr Asn Pro Lys Leu Cys Leu Ser
       450                 455                 460
   Glu Ile His Lys Met Glu Glu Val Ser Gly Thr Lys Gly Arg Gln Glu
   465                 470                 475                 480
   Arg Asn Asp Ile Ala Leu Lys Thr Asn Gly Asp Gln Ala Ser Cys Glu
                   485                 490                 495
   Asn Glu Leu Leu Lys Phe Ser Tyr Ile Arg Thr Ser Phe Asp Lys Ile
               500                 505                 510
   Leu Leu Arg Trp Glu Pro Tyr Trp Pro Pro Asp Phe Arg Asp Leu Leu
           515                 520                 525
   Gly Phe Met Leu Phe Tyr Lys Glu Ala Pro Tyr Gln Asn Val Thr Glu
       530                 535                 540
   Phe Asp Gly Gln Asp Ala Cys Gly Ser Asn Ser Trp Thr Val Val Asp
   545                 550                 555                 560
   Ile Asp Pro Pro Leu Arg Ser Asn Asp Pro Lys Ser Gln Asn His Pro
                   565                 570                 575
   Gly Trp Leu Met Arg Gly Leu Lys Pro Trp Thr Gln Tyr Ala Ile Phe
               580                 585                 590
   Val Lys Thr Leu Val Thr Phe Ser Asp Glu Arg Arg Thr Tyr Gly Ala
           595                 600                 605
   Lys Ser Asp Ile Ile Tyr Val Gln Thr Asp Ala Thr Asn Pro Ser Val
       610                 615                 620
   Pro Leu Asp Pro Ile Ser Val Ser Asn Ser Ser Ser Gln Ile Ile Leu
   625                 630                 635                 640
   Lys Trp Lys Pro Pro Ser Asp Pro Asn Gly Asn Ile Thr His Tyr Leu
                   645                 650                 655
   Val Phe Trp Glu Arg Gln Ala Glu Asp Ser Glu Leu Phe Glu Leu Asp
               660                 665                 670
   Tyr Cys Leu Lys Gly Leu Lys Leu Pro Ser Arg Thr Trp Ser Pro Pro
           675                 680                 685
   Phe Glu Ser Glu Asp Ser Gln Lys His Asn Gln Ser Glu Tyr Glu Asp
       690                 695                 700
   Ser Ala Gly Glu Cys Cys Ser Cys Pro Lys Thr Asp Ser Gln Ile Leu
   705                 710                 715                 720
   Lys Glu Leu Glu Glu Ser Ser Phe Arg Lys Thr Phe Glu Asp Tyr Leu
                   725                 730                 735
   His Asn Val Val Phe Val Pro Arg Pro Ser Arg Lys Arg Arg Ser Leu
               740                 745                 750
   Gly Asp Val Gly Asn Val Thr Val Ala Val Pro Thr Val Ala Ala Phe
           755                 760                 765
   Pro Asn Thr Ser Ser Thr Ser Val Pro Thr Ser Pro Glu Glu His Arg
       770                 775                 780
   Pro Phe Glu Lys Val Val Asn Lys Glu Ser Leu Val Ile Ser Gly Leu
   785                 790                 795                 800
   Arg His Phe Thr Gly Tyr Arg Ile Glu Leu Gln Ala Cys Asn Gln Asp
                   805                 810                 815
   Thr Pro Glu Glu Arg Cys Ser Val Ala Ala Tyr Val Ser Ala Arg Thr
               820                 825                 830
   Met Pro Glu Ala Lys Ala Asp Asp Ile Val Gly Pro Val Thr His Glu
           835                 840                 845
   Ile Phe Glu Asn Asn Val Val His Leu Met Trp Gln Glu Pro Lys Glu
       850                 855                 860
   Pro Asn Gly Leu Ile Val Leu Tyr Glu Val Ser Tyr Arg Arg Tyr Gly
   865                 870                 875                 880
   Asp Glu Glu Leu His Leu Cys Val Ser Arg Lys His Phe Ala Leu Glu
                   885                 890                 895
   Arg Gly Cys Arg Leu Arg Gly Leu Ser Pro Gly Asn Tyr Ser Val Arg
               900                 905                 910
   Ile Arg Ala Thr Ser Leu Ala Gly Asn Gly Ser Trp Thr Glu Pro Thr
           915                 920                 925
   Tyr Phe Tyr Val Thr Asp Tyr Leu Asp Val Pro Ser Asn Ile Ala Lys
       930                 935                 940
   Ile Ile Ile Gly Pro Leu Ile Phe Val Phe Leu Phe Ser Val Val Ile
   945                 950                 955                 960
   Gly Ser Ile Tyr Leu Phe Leu Arg Lys Arg Gln Pro Asp Gly Pro Leu
                   965                 970                 975
   Gly Pro Leu Tyr Ala Ser Ser Asn Pro Glu Tyr Leu Ser Ala Ser Asp
               980                 985                 990
   Val Phe Pro Cys Ser Val Tyr Val Pro Asp Glu Trp Glu Val Ser Arg
           995                 1000                1005
   Glu Lys Ile Thr Leu Leu Arg Glu Leu Gly Gln Gly Ser Phe Gly Met
       1010                1015                1020
   Val Tyr Glu Gly Asn Ala Arg Asp Ile Ile Lys Gly Glu Ala Glu Thr
   1025                1030                1035                1040
   Arg Val Ala Val Lys Thr Val Asn Glu Ser Ala Ser Leu Arg Glu Arg
                       1045                1050                1055
   Ile Glu Phe Leu Asn Glu Ala Ser Val Met Lys Gly Phe Thr Cys His
                   1060                1065                1070
   His Val Val Arg Leu Leu Gly Val Val Ser Lys Gly Gln Pro Thr Leu
               1075                1080                1085
   Val Val Met Glu Leu Met Ala His Gly Asp Leu Lys Ser Tyr Leu Arg
           1090                1095                1100
   Ser Leu Arg Pro Glu Ala Glu Asn Asn Pro Gly Arg Pro Pro Pro Thr
   1105                1110                1115                1120
   Leu Gln Glu Met Ile Gln Met Ala Ala Glu Ile Ala Asp Gly Met Ala
                       1125                1130                1135
   Tyr Leu Asn Ala Lys Lys Phe Val His Arg Asp Leu Ala Ala Arg Asn
                   1140                1145                1150
   Cys Met Val Ala His Asp Phe Thr Val Lys Ile Gly Asp Phe Gly Met
               1155                1160                1165
   Thr Arg Asp Ile Tyr Glu Thr Asp Tyr Tyr Arg Lys Gly Gly Lys Gly
           1170                1175                1180
   Leu Leu Pro Val Arg Trp Met Ala Pro Glu Ser Leu Lys Asp Gly Val
   1185                1190                1195                1200
   Phe Thr Thr Ser Ser Asp Met Trp Ser Phe Gly Val Val Leu Trp Glu
                       1205                1210                1215
   Ile Thr Ser Leu Ala Glu Gln Pro Tyr Gln Gly Leu Ser Asn Glu Gln
                   1220                1225                1230
   Val Leu Lys Phe Val Met Asp Gly Gly Tyr Leu Asp Gln Pro Asp Asn
               1235                1240                1245
   Cys Pro Glu Arg Val Thr Asp Leu Met Arg Met Cys Trp Gln Phe Asn
           1250                1255                1260
   Pro Lys Met Arg Pro Thr Phe Leu Glu Ile Val Asn Leu Leu Lys Asp
   1265                1270                1275                1280
   Asp Leu His Pro Ser Phe Pro Glu Val Ser Phe Phe His Ser Glu Glu
                       1285                1290                1295
   Asn Lys Ala Pro Glu Ser Glu Glu Leu Glu Met Glu Phe Glu Asp Met
                   1300                1305                1310
   Glu Asn Val Pro Leu Asp Arg Ser Ser His Cys Gln Arg Glu Glu Ala
               1315                1320                1325
   Gly Gly Arg Asp Gly Gly Ser Ser Leu Gly Phe Lys Arg Ser Tyr Glu
           1330                1335                1340
   Glu His Ile Pro Tyr Thr His Met Asn Gly Gly Lys Lys Asn Gly Arg
   1345                1350                1355                1360
   Ile Leu Thr Leu Pro Arg Ser Asn Pro Ser Gln Leu Val Thr Asp Ala
                       1365                1370                1375
   Lys Asn Ile Lys Lys Gly Pro Ala Pro Phe Tyr Pro Leu Glu Asp Gly
                   1380                1385                1390
   Thr Ala Gly Glu Gln Leu His Lys Ala Met Lys Arg Tyr Ala Leu Val
               1395                1400                1405
   Pro Gly Thr Ile Ala Phe Thr Asp Ala His Ile Glu Val Asp Ile Thr
           1410                1415                1420
   Tyr Ala Glu Tyr Phe Glu Met Ser Val Arg Leu Ala Glu Ala Met Lys
   1425                1430                1435                1440
   Arg Tyr Gly Leu Asn Thr Asn His Arg Ile Val Val Cys Ser Glu Asn
                       1445                1450                1455
   Ser Leu Gln Phe Phe Met Pro Val Leu Gly Ala Leu Phe Ile Gly Val
                   1460                1465                1470
   Ala Val Ala Pro Ala Asn Asp Ile Tyr Asn Glu Arg Glu Leu Leu Asn
               1475                1480                1485
   Ser Met Gly Ile Ser Gln Pro Thr Val Val Phe Val Ser Lys Lys Gly
           1490                1495                1500
   Leu Gln Lys Ile Leu Asn Val Gln Lys Lys Leu Pro Ile Ile Gln Lys
   1505                1510                1515                1520
   Ile Ile Ile Met Asp Ser Lys Thr Asp Tyr Gln Gly Phe Gln Ser Met
                       1525                1530                1535
   Tyr Thr Phe Val Thr Ser His Leu Pro Pro Gly Phe Asn Glu Tyr Asp
                   1540                1545                1550
   Phe Val Pro Glu Ser Phe Asp Arg Asp Lys Thr Ile Ala Leu Ile Met
           1555                1560                1565
   Asn Ser Ser Gly Ser Thr Gly Leu Pro Lys Gly Val Ala Leu Pro His
       1570                1575                1580
   Arg Thr Ala Cys Val Arg Phe Ser His Ala Arg Asp Pro Ile Phe Gly
   1585                1590                1595                1600
   Asn Gln Ile Ile Pro Asp Thr Ala Ile Leu Ser Val Val Pro Phe His
                   1605                1610                1615
   His Gly Phe Gly Met Phe Thr Thr Leu Gly Tyr Leu Ile Cys Gly Phe
               1620                1625                1630
   Arg Val Val Leu Met Tyr Arg Phe Glu Glu Glu Leu Phe Leu Arg Ser
           1635                1640                1645
   Leu Gln Asp Tyr Lys Ile Gln Ser Ala Leu Leu Val Pro Thr Leu Phe
       1650                1655                1660
   Ser Phe Phe Ala Lys Ser Thr Leu Ile Asp Lys Tyr Asp Leu Ser Asn
   1665                1670                1675                1680
   Leu His Glu Ile Ala Ser Gly Gly Ala Pro Leu Ser Lys Glu Val Gly
                   1685                1690                1695
   Glu Ala Val Ala Lys Arg Phe His Leu Pro Gly Ile Arg Gln Gly Tyr
               1700                1705                1710
   Gly Leu Thr Glu Thr Thr Ser Ala Ile Leu Ile Thr Pro Glu Gly Asp
           1715                1720                1725
   Asp Lys Pro Gly Ala Val Gly Lys Val Val Pro Phe Phe Glu Ala Lys
       1730                1735                1740
   Val Val Asp Leu Asp Thr Gly Lys Thr Leu Gly Val Asn Gln Arg Gly
   1745                1750                1755                1760
   Glu Leu Cys Val Arg Gly Pro Met Ile Met Ser Gly Tyr Val Asn Asn
                   1765                1770                1775
   Pro Glu Ala Thr Asn Ala Leu Ile Asp Lys Asp Gly Trp Leu His Ser
               1780                1785                1790
   Gly Asp Ile Ala Tyr Trp Asp Glu Asp Glu His Phe Phe Ile Val Asp
           1795                1800                1805
   Arg Leu Lys Ser Leu Ile Lys Tyr Lys Gly Tyr Gln Val Ala Pro Ala
       1810                1815                1820
   Glu Leu Glu Ser Ile Leu Leu Gln His Pro Asn Ile Phe Asp Ala Gly
   1825                1830                1835                1840
   Val Ala Gly Leu Pro Asp Asp Asp Ala Gly Glu Leu Pro Ala Ala Val
                   1845                1850                1855
   Val Val Leu Glu His Gly Lys Thr Met Thr Glu Lys Glu Ile Val Asp
               1860                1865                1870
   Tyr Val Ala Ser Gln Val Thr Thr Ala Lys Lys Leu Arg Gly Gly Val
           1875                1880                1885
   Val Phe Val Asp Glu Val Pro Lys Gly Leu Thr Gly Lys Leu Asp Ala
       1890                1895                1900
   Arg Lys Ile Arg Glu Ile Leu Ile Lys Ala Lys Lys Gly Gly Lys Ile
   1905                1910                1915                1920
   Ala Val
<210> 15
   <211> 2781
   <212> DNA
   <213> Artificial Sequence
<220>
   <221> source
   <222> 1..2781
   <223> /organism="Artificial Sequence" /note="extracellular domain of human IGF1R" /mol_type="unassigned DNA"
<400> 15
   atgaagtctg gctccggagg agggtccccg acctcgctgt gggggctcct gtttctctcc 60
   gccgcgctct cgctctggcc gacgagtgga gaaatctgcg ggccaggcat cgacatccgc 120
   aacgactatc agcagctgaa gcgcctggag aactgcacgg tgatcgaggg ctacctccac 180
   atcctgctca tctccaaggc cgaggactac cgcagctacc gcttccccaa gctcacggtc 240
   attaccgagt acttgctgct gttccgagtg gctggcctcg agagcctcgg agacctcttc 300
   cccaacctca cggtcatccg cggctggaaa ctcttctaca actacgccct ggtcatcttc 360
   gagatgacca atctcaagga tattgggctt tacaacctga ggaacattac tcggggggcc 420
   atcaggattg agaaaaatgc tgacctctgt tacctctcca ctgtggactg gtccctgatc 480
   ctggatgcgg tgtccaataa ctacattgtg gggaataagc ccccaaagga atgtggggac 540
   ctgtgtccag ggaccatgga ggagaagccg atgtgtgaga agaccaccat caacaatgag 600
   tacaactacc gctgctggac cacaaaccgc tgccagaaaa tgtgcccaag cacgtgtggg 660
   aagcgggcgt gcaccgagaa caatgagtgc tgccaccccg agtgcctggg cagctgcagc 720
   gcgcctgaca acgacacggc ctgtgtagct tgccgccact actactatgc cggtgtctgt 780
   gtgcctgcct gcccgcccaa cacctacagg tttgagggct ggcgctgtgt ggaccgtgac 840
   ttctgcgcca acatcctcag cgccgagagc agcgactccg aggggtttgt gatccacgac 900
   ggcgagtgca tgcaggagtg cccctcgggc ttcatccgca acggcagcca gagcatgtac 960
   tgcatccctt gtgaaggtcc ttgcccgaag gtctgtgagg aagaaaagaa aacaaagacc 1020
   attgattctg ttacttctgc tcagatgctc caaggatgca ccatcttcaa gggcaatttg 1080
   ctcattaaca tccgacgggg gaataacatt gcttcagagc tggagaactt catggggctc 1140
   atcgaggtgg tgacgggcta cgtgaagatc cgccattctc atgccttggt ctccttgtcc 1200
   ttcctaaaaa accttcgcct catcctagga gaggagcagc tagaagggaa ttactccttc 1260
   tacgtcctcg acaaccagaa cttgcagcaa ctgtgggact gggaccaccg caacctgacc 1320
   atcaaagcag ggaaaatgta ctttgctttc aatcccaaat tatgtgtttc cgaaatttac 1380
   cgcatggagg aagtgacggg gactaaaggg cgccaaagca aaggggacat aaacaccagg 1440
   aacaacgggg agagagcctc ctgtgaaagt gacgtcctgc atttcacctc caccaccacg 1500
   tcgaagaatc gcatcatcat aacctggcac cggtaccggc cccctgacta cagggatctc 1560
   atcagcttca ccgtttacta caaggaagca ccctttaaga atgtcacaga gtatgatggg 1620
   caggatgcct gcggctccaa cagctggaac atggtggacg tggacctccc gcccaacaag 1680
   gacgtggagc ccggcatctt actacatggg ctgaagccct ggactcagta cgccgtttac 1740
   gtcaaggctg tgaccctcac catggtggag aacgaccata tccgtggggc caagagtgag 1800
   atcttgtaca ttcgcaccaa tgcttcagtt ccttccattc ccttggacgt tctttcagca 1860
   tcgaactcct cttctcagtt aatcgtgaag tggaaccctc cctctctgcc caacggcaac 1920
   ctgagttact acattgtgcg ctggcagcgg cagcctcagg acggctacct ttaccggcac    1980
   aattactgct ccaaagacaa aatccccatc aggaagtatg ccgacggcac catcgacatt    2040
   gaggaggtca cagagaaccc caagactgag gtgtgtggtg gggagaaagg gccttgctgc    2100
   gcctgcccca aaactgaagc cgagaagcag gccgagaagg aggaggctga ataccgcaaa    2160
   gtctttgaga atttcctgca caactccatc ttcgtgccca gacctgaaag gaagcggaga    2220
   gatgtcatgc aagtggccaa caccaccatg tccagccgaa gcaggaacac cacggccgca    2280
   gacacctaca acatcaccga cccggaagag ctggagacag agtacccttt ctttgagagc    2340
   agagtggata acaaggagag aactgtcatt tctaaccttc ggcctttcac attgtaccgc    2400
   atcgatatcc acagctgcaa ccacgaggct gagaagctgg gctgcagcgc ctccaacttc    2460
   gtctttgcaa ggactatgcc cgcagaagga gcagatgaca ttcctgggcc agtgacctgg    2520
   gagccaaggc ctgaaaactc catcttttta aagtggccgg aacctgagaa tcccaatgga    2580
   ttgattctaa tgtatgaaat aaaatacgga tcacaagttg aggatcagcg agaatgtgtg    2640
   tccagacagg aatacaggaa gtatggaggg gccaagctaa accggctaaa cccggggaac    2700
   tacacagccc ggattcaggc cacatctctc tctgggaatg ggtcgtggac agatcctgtg    2760
   ttcttctatg tccaggccaa a                                              2781
<210> 16
   <211> 927
   <212> PRT
   <213> Artificial Sequence
<220>
   <223> extracellular domain of human IGF1R
<400> 16
   Met Lys Ser Gly Ser Gly Gly Gly Ser Pro Thr Ser Leu Trp Gly Leu
   1               5                   10                  15
   Leu Phe Leu Ser Ala Ala Leu Ser Leu Trp Pro Thr Ser Gly Glu Ile
               20                  25                  30
   Cys Gly Pro Gly Ile Asp Ile Arg Asn Asp Tyr Gln Gln Leu Lys Arg
           35                  40                  45
   Leu Glu Asn Cys Thr Val Ile Glu Gly Tyr Leu His Ile Leu Leu Ile
       50                  55                  60
   Ser Lys Ala Glu Asp Tyr Arg Ser Tyr Arg Phe Pro Lys Leu Thr Val
   65                  70                  75                  80
   Ile Thr Glu Tyr Leu Leu Leu Phe Arg Val Ala Gly Leu Glu Ser Leu
                   85                  90                  95
   Gly Asp Leu Phe Pro Asn Leu Thr Val Ile Arg Gly Trp Lys Leu Phe
               100                 105                 110
   Tyr Asn Tyr Ala Leu Val Ile Phe Glu Met Thr Asn Leu Lys Asp Ile
           115                 120                 125
   Gly Leu Tyr Asn Leu Arg Asn Ile Thr Arg Gly Ala Ile Arg Ile Glu
       130                 135                 140
   Lys Asn Ala Asp Leu Cys Tyr Leu Ser Thr Val Asp Trp Ser Leu Ile
   145                 150                 155                 160
   Leu Asp Ala Val Ser Asn Asn Tyr Ile Val Gly Asn Lys Pro Pro Lys
                   165                 170                 175
   Glu Cys Gly Asp Leu Cys Pro Gly Thr Met Glu Glu Lys Pro Met Cys
                   180                     185                     190
   Glu Lys Thr Thr Ile Asn Asn Glu Tyr Asn Tyr Arg Cys Trp Thr Thr
               195                     200                     205
   Asn Arg Cys Gln Lys Met Cys Pro Ser Thr Cys Gly Lys Arg Ala Cys
       210                     215                     220
   Thr Glu Asn Asn Glu Cys Cys His Pro Glu Cys Leu Gly Ser Cys Ser
   225                     230                     235                     240
   Ala Pro Asp Asn Asp Thr Ala Cys Val Ala Cys Arg His Tyr Tyr Tyr
                       245                     250                     255
   Ala Gly Val Cys Val Pro Ala Cys Pro Pro Asn Thr Tyr Arg Phe Glu
                   260                     265                     270
   Gly Trp Arg Cys Val Asp Arg Asp Phe Cys Ala Asn Ile Leu Ser Ala
               275                     280                     285
   Glu Ser Ser Asp Ser Glu Gly Phe Val Ile His Asp Gly Glu Cys Met
       290                     295                     300
   Gln Glu Cys Pro Ser Gly Phe Ile Arg Asn Gly Ser Gln Ser Met Tyr
   305                     310                     315                     320
   Cys Ile Pro Cys Glu Gly Pro Cys Pro Lys Val Cys Glu Glu Glu Lys
                       325                     330                     335
   Lys Thr Lys Thr Ile Asp Ser Val Thr Ser Ala Gln Met Leu Gln Gly
                   340                     345                     350
   Cys Thr Ile Phe Lys Gly Asn Leu Leu Ile Asn Ile Arg Arg Gly Asn
               355                     360                     365
   Asn Ile Ala Ser Glu Leu Glu Asn Phe Met Gly Leu Ile Glu Val Val
       370                     375                     380
   Thr Gly Tyr Val Lys Ile Arg His Ser His Ala Leu Val Ser Leu Ser
   385                     390                     395                     400
   Phe Leu Lys Asn Leu Arg Leu Ile Leu Gly Glu Glu Gln Leu Glu Gly
                       405                     410                     415
   Asn Tyr Ser Phe Tyr Val Leu Asp Asn Gln Asn Leu Gln Gln Leu Trp
                   420                     425                     430
   Asp Trp Asp His Arg Asn Leu Thr Ile Lys Ala Gly Lys Met Tyr Phe
               435                     440                     445
   Ala Phe Asn Pro Lys Leu Cys Val Ser Glu Ile Tyr Arg Met Glu Glu
       450                     455                     460
   Val Thr Gly Thr Lys Gly Arg Gln Ser Lys Gly Asp Ile Asn Thr Arg
   465                     470                     475                     480
   Asn Asn Gly Glu Arg Ala Ser Cys Glu Ser Asp Val Leu His Phe Thr
                       485                     490                     495
   Ser Thr Thr Thr Ser Lys Asn Arg Ile Ile Ile Thr Trp His Arg Tyr
                   500                     505                     510
   Arg Pro Pro Asp Tyr Arg Asp Leu Ile Ser Phe Thr Val Tyr Tyr Lys
               515                     520                     525
   Glu Ala Pro Phe Lys Asn Val Thr Glu Tyr Asp Gly Gln Asp Ala Cys
       530                     535                     540
   Gly Ser Asn Ser Trp Asn Met Val Asp Val Asp Leu Pro Pro Asn Lys
   545                     550                     555                     560
   Asp Val Glu Pro Gly Ile Leu Leu His Gly Leu Lys Pro Trp Thr Gln
                       565                     570                     575
   Tyr Ala Val Tyr Val Lys Ala Val Thr Leu Thr Met Val Glu Asn Asp
                   580                     585                     590
   His Ile Arg Gly Ala Lys Ser Glu Ile Leu Tyr Ile Arg Thr Asn Ala
               595                     600                     605
   Ser Val Pro Ser Ile Pro Leu Asp Val Leu Ser Ala Ser Asn Ser Ser
       610                     615                     620
   Ser Gln Leu Ile Val Lys Trp Asn Pro Pro Ser Leu Pro Asn Gly Asn
   625                     630                     635                     640
   Leu Ser Tyr Tyr Ile Val Arg Trp Gln Arg Gln Pro Gln Asp Gly Tyr
                       645                     650                     655
   Leu Tyr Arg His Asn Tyr Cys Ser Lys Asp Lys Ile Pro Ile Arg Lys
                   660                     665                     670
   Tyr Ala Asp Gly Thr Ile Asp Ile Glu Glu Val Thr Glu Asn Pro Lys
               675                     680                     685
   Thr Glu Val Cys Gly Gly Glu Lys Gly Pro Cys Cys Ala Cys Pro Lys
       690                 695                 700
   Thr Glu Ala Glu Lys Gln Ala Glu Lys Glu Glu Ala Glu Tyr Arg Lys
   705                 710                 715                 720
   Val Phe Glu Asn Phe Leu His Asn Ser Ile Phe Val Pro Arg Pro Glu
                   725                 730                 735
   Arg Lys Arg Arg Asp Val Met Gln Val Ala Asn Thr Thr Met Ser Ser
               740                 745                 750
   Arg Ser Arg Asn Thr Thr Ala Ala Asp Thr Tyr Asn Ile Thr Asp Pro
           755                 760                 765
   Glu Glu Leu Glu Thr Glu Tyr Pro Phe Phe Glu Ser Arg Val Asp Asn
       770                 775                 780
   Lys Glu Arg Thr Val Ile Ser Asn Leu Arg Pro Phe Thr Leu Tyr Arg
   785                 790                 795                 800
   Ile Asp Ile His Ser Cys Asn His Glu Ala Glu Lys Leu Gly Cys Ser
                   805                 810                 815
   Ala Ser Asn Phe Val Phe Ala Arg Thr Met Pro Ala Glu Gly Ala Asp
               820                 825                 830
   Asp Ile Pro Gly Pro Val Thr Trp Glu Pro Arg Pro Glu Asn Ser Ile
           835                 840                 845
   Phe Leu Lys Trp Pro Glu Pro Glu Asn Pro Asn Gly Leu Ile Leu Met
       850                 855                 860
   Tyr Glu Ile Lys Tyr Gly Ser Gln Val Glu Asp Gln Arg Glu Cys Val
   865                 870                 875                 880
   Ser Arg Gln Glu Tyr Arg Lys Tyr Gly Gly Ala Lys Leu Asn Arg Leu
                   885                 890                 895
   Asn Pro Gly Asn Tyr Thr Ala Arg Ile Gln Ala Thr Ser Leu Ser Gly
               900                 905                 910
   Asn Gly Ser Trp Thr Asp Pro Val Phe Phe Tyr Val Gln Ala Lys
           915                 920                 925
<210> 17
   <211> 33
   <212> DNA
   <213> Artificial Sequence
<220>
   <221> source
   <222> 1..33
   <223> /organism="Artificial Sequence" /note="Primer P5" /mol_type="unassigned DNA"
<400> 17
   acggttaaca tgggcaccgg gggccggcgg ggg 33
<210> 18
   <211> 33
   <212> DNA
   <213> Artificial Sequence
<220>
   <221> source
   <222> 1..33
   <223> /organism="Artificial Sequence" /note="Primer P6" /mol_type="unassigned DNA"
<400> 18
   acgcaattgc agcaagatct tgtcaaaaga tgt 33
<210> 19
   <211> 36
   <212> DNA
   <213> Artificial Sequence
<220>
   <221> source
   <222> 1..36
   <223> /organism="Artificial Sequence" /note="Primer P7" /mol_type="unassigned DNA"
<400> 19
   atcggatcca ccatgaagtc tggctccgga ggaggg 36
<210> 20
   <211> 83
   <212> DNA
   <213> Artificial Sequence
<220>
   <221> source
   <222> 1..83
   <223> /organism="Artificial Sequence" /note="Primer P8" /mol_type="unassigned DNA"
<400> 20
   atcaagcttc actttcctgc tcctggttgc tgattctctg aaagctcact gctgccaagt      60
   ttggcctgga catagaagaa cac                                              83

## Description

The present invention relates to methods useful in the identification of antibodies reactive with one or more members of the insulin receptor family.

### Background of the invention

The insulin receptor family (IRF) mainly consists of four receptors that bind insulin and/or IGF1 with different affinities: insulin receptor (IR), IGF1 receptor (IGF1R), and IGF2 receptor (IGF2R), and the insulin-IGFl hybrid receptor (IIHR). Another orphan member of this family is the insulin receptor-related receptor (IRRR).

From the above mentioned receptors, IR, IGF1R, IIHR, and IRRR belong to the family of ligand-activated tyrosine kinase receptors, whereas IGF2R is a transmembrane monomer with a large extracellular domain and no intrinsic signaling capabilities.

IR and IGF1R are expressed at the cell surface as homodimers composed of two identical monomers, or as heterodimers composed of two different receptor monomers (e.g., the insulin-IGFl hybrid receptor (IIHR)). IIHRs are widely distributed in different mammalian tissues. They behave in a manner similar to IGF1R with respect to ligand-induced tyrosine phosphorylation signaling.

Both IGF1R and IR receptors are composed of two alpha and two beta glycosylated subunits, wherein two transmembrane alpha-beta chains form together a disulfide-linked heterotetramer (beta-alpha-alpha-beta). The receptors have an extracellular ligand binding domain, a single transmembrane domain, and a cytoplasmic domain exhibiting the tyrosine kinase activity. The extracellular domain is composed of the entire alpha subunits and a portion of the N-terminus of the beta subunits, while the intracellular portion of the beta subunits contains the tyrosine kinase (TK) domain.

While similar in structure, IGF1R and IR serve different physiological functions. IR is primarily involved in metabolic functions, whereas IGF1R mediates growth and differentiation. However, their ligands insulin and IGF-1 can induce both mitogenic and metabolic effects when binding the receptors.

Due to the structural relationship of the members of the IRF, the ligand insulin does not only bind to IR, but also to IGF1R and IIHR, the ligand IGF-1 does not only bind to IGF1R, but also to IR, IIHR, and IGF2R, and the ligand IGF-2 does not only bind to IGF2R, but also to IR, IIHR, and IGF1R.

Autoantibodies are known to play an important role in the generation of autoimmune disorders. For example, the presence of autoantibodies to IR in a subject indicates the onset of diabetes (Type B insulin resistance).

Furthermore, non-endogenous antibodies to IGF1R are currently in development to provide novel therapeutical approaches in the treatment of cancer. For example, the drug candidate's figitumumab, developed by Pfizer Inc. and dalatozumab, developed by Merck & Co Inc. are monoclonal therapeutical antibodies to IGF1R, which are currently investigated for the treatment of various types of cancer. For example, patent applications WO 2002/053596 A, WO 2004/083248 A, WO 2005/016967 A, WO 2005/016970 A, US 2005/0249730, US 2005/0084906, WO 2005/052005 A, WO 2005/058967 A, WO 2005/094376 A, WO 2006/008639 A, WO 2006/013472 A, and WO 2008/077546 A refer to the treatment of cancer by means of anti-IGFI R antibodies.

Masseyeff RF (editor-in-chief), Albert, WH and Staines NA, Methods in Immunologic Analysis, Vol 1: Fundamentals (1993) VCh Weinheim, pp. 475-490 teach the basic principles of a dual-antigen sandwich immunoassay.

The prevalence and role of autoantibodies which are capable to bind members of the IRF is not yet fully understood and requires further investigation to successfully allow diagnosis, prevention, and treatment of autoimmune disorders (such as, e.g., insulin dependent diabetes mellitus (IDDM), Morbus Basedow, Graves Orbitopathy), obesity, neurological disorders, growth disorders, cancer generation and development (including breast, colon, ovarian, prostate, lung), and other cellular proliferative disorders and other diseases or dysfunctions. For example, dysfunctional IR signaling has been reported in disorders including diabetes type I and II, dementia, and cancer.

More specific and more sensitive assay methods may be helpful to develop a deeper understanding of these complex pathways and will serve as valuable tools in the diagnosis and research in this context, e.g., by allowing discrimination of different physiological states between a healthy individual and progressing dysfunctions. In particular, the measurement of the autoantibodies to IRF members as serological markers can be useful in the early diagnosis or differential diagnosis, in prevention and/or therapy of any of the above mentioned autoimmune disorders.

Furthermore, the improvements of present invention are enabling the identification of modulators (i.e. activators, inhibitors or otherwise affecting or triggering molecules) of the interaction of the members of the IRF and analyte antibodies that affect or trigger the receptor activity, and which therefore may be suitable as pharmaceutically effective compounds in the diagnosis, prevention and/or treatment of any of the diseases which are related to members of the IRF.

Expression of a recombinant human IGF1R-luciferase fusion protein in stably transfected HEK293 cells has been shown by W. Minich et al. (Detection of Autoantibodies to the Insulin-Like Growth Factor-1 Receptor in Patients with Graves Orbitopathy by Luminescent Immunoprecipitation Analysis. Poster Presentation ITC 2010 Paris) who showed quantification of auto-antibodies to IGF1R in sera of patients with Graves' orbitopathy using an immunoprecipitation assay (IPA).

Jin K.-C. et al. (Bioanalysis September 2011, 3(18): 2107-2117) reported the development of two ELISA assays for the quantification of therapeutical anti-IGFIR antibodies in order to study their pharmacokinetic properties. These two assays are based on classical ELISA methods using labeled anti-human IgG antibodies or labeled anti-human IgGFc antibodies as the detecting reagent.

Furthermore, an ELISA Kit for human anti-insulin receptor antibody detection is provided by Uscn Life Science Inc. (Wuhan, CN). This kit is based on the principle of a sandwich enzyme immunoassay, wherein a microtiter plate has been pre-coated with the antigen. After incubation with the sample, biotin-conjugated antibody is added, and HRP-conjugated avidin is used as the detecting reagent.

Generally, it is known that the use of labeled anti-IgG- or anti-IgGFc-antibodies as the detecting reagent may form complexes with unspecifically antigen-bound antibodies, thereby resulting in false positive signals. Similarly, the biotin-avidin amplification technique is known to result in poor signal to noise ratios. Therefore, there is still the need for assay technology and performance improvement, for example, in the area of high throughput screening (HTS-) assays, where sample size is often critical and both consumption of sample volumes and assay reagents need always further reduction due to limited availabilities and the requirement of cost reduction. Moreover, epidemiological analyses or HTS-assays profit from minimal reaction step numbers, low hands-on-time during assay handling and other technical improvements.

Here, the present invention, overcomes the aforementioned problems by providing improved detection methods employed in the analysis and screening of antibodies reactive with antigenic molecules which are selected from the IRF. In particular, by providing the new immunoassay methods, the present invention surprisingly improves specificity and sensitivity of prior art antibody detection as discussed above. The use of the methods according to the invention is much closer to the physiological conditions and therefore allows the antigenic molecules to expose their correct three-dimensional structure. Consequently, the methods according to the present invention are not only favorable for performing automated assay formats in research and clinical laboratories but are more sensitive and specific than prior art assays.

Surprisingly, it was found by means of the methods according to the present invention that autoantibody-positive samples do cross-react with different members of the IRF. Due to the surprisingly improved sensitivity and specificity of the methods according to the present invention, the new assay format allows for a more differentiated investigation and development of novel therapeutics and diagnostic tools in the prophylaxis and treatment of disorders and diseases related to members of the IRF. Furthermore, it is possible to perform the method according to the invention with any kind of a bivalent antibody suitable according to the present invention regardless from which source the antibody is derived from.

### Summary of the Invention

Therefore, it is one embodiment of the present invention to provide a method for the measurement of autoantibodies to IRF members as serological markers comprising detecting in a sample to be investigated the presence and/or the binding properties of analyte antibodies reactive with one or more antigenic molecules, said method comprising: (a) providing one or more first antigenic molecules with which analyte antibodies when present in said sample can interact and which first antigenic molecule is selected from the insulin receptor family (IRF); and (b) providing one or more second antigenic molecules with which analyte antibodies when present in said sample can interact and which second antigenic molecule is selected from the IRF; and (c) contacting said first antigenic molecules as provided by step (a) and said second antigenic molecules as provided by step (b) simultaneously or successively with the sample to be investigated, whereby analyte antibodies when present in said sample can interact with said antigenic molecules so as to form complexes comprising [first antigenic molecule]-[analyte antibody]-[second antigenic molecule]; and (d) prior to, or concurrent with, or subsequent to, step (c), providing immobilizing means whereby said first antigenic molecule as present in the said complexes formed in step (c), respectively, as capable to form complexes in step (c) is immobilized to a solid support prior to, or concurrent with, or subsequent to, step (c); and (e) prior to, or concurrent with, or subsequent to, step (c), providing first labeling means whereby said second antigenic molecule as present in the said complexes formed in step (c), respectively, as capable to form complexes in step (c) is labeled with said first labeling means prior to, or concurrent with, or subsequent to, step (c); and (g) detecting the presence of complexes formed in or subsequent to step (c) so as to provide indication of analyte antibodies present in said sample. Furthermore, the said method as disclosed can be used for the identification of a modulator of the binding properties of the said analyte antibodies reactive with one or more antigenic molecules in combination with the general knowledge of the person skilled in the art of screening for active compounds with the potential for use as a pharmaceutically active compound.

The methods according to the present invention are, in vitro methods. Moreover, they may comprise steps in addition to those explicitly mentioned above including sample pretreatments or evaluation of the results (e.g., with respect to the detection of the presence of complexes formed) obtained by the methods. The methods may be carried out manually and/or assisted by automation. Preferably, one or more of steps (a), (b), (c), (d), (e), (f) and/or (g) may in total or in part be assisted by automation including suitable robotic and sensory equipment for detection and/or a computer-implemented processing and/or analysis in step (g). For a person skilled in the art, it is known that the methods according to the present invention require calibration or standardization to compare the detected signals, e.g. of the presence of complexes with (one or more) known amounts of reference complex formation.

In other embodiments of the present invention the said first antigenic molecules and the said second antigenic molecules are identical or, alternatively, they are not identical (i.e. different). In other embodiments of the present invention the said first antigenic molecules and the said second antigenic molecules are not identical and belong to different members of the IRF. In other embodiments of the present invention the said first antigenic molecules and/or the said second antigenic molecules are embedded in a membrane environment. In still other embodiments the methods of the present invention allow the detection of an antibody against a member of the IRF of about 3 ng/ml, preferably of about 1 ng/ml, more preferred of about 0,3 ng/ml, even more preferred of about 0,1 ng/ml and most preferred of about 0,03 ng/ml.

In other embodiments of the methods of the present invention the one or more first antigenic molecules, which are immobilized to a solid support, are provided prior to step (c) of the method of detecting autoantibodies according to the present invention.

The one or more first antigenic molecules may be immobilized to a solid support prior to contact with a sample to be investigated. Optionally, the solid support may be provided in a liquid phase (e.g., dispersion, suspension, and colloid). Such immobilized one or more first antigenic molecules are subsequently contacted with the said sample either simultaneously or successively with contact of the said sample and with one or more second antigenic molecules. The immobilized one or more first antigenic molecules when contacted with the said sample may form intermediate complexes comprising [first antigenic molecule]-[analyte antibody] wherein the one or more first antigenic molecule is immobilized to a solid support and the thus formed immobilized intermediate complex is subsequently contacted with the one or more second antigenic molecules, present in solution, so as to form the hitherto described complexes comprising [first antigenic molecule]-[analyte antibody]-[second antigenic molecule] directly or indirectly immobilized to a solid support via the first antigenic molecule.

In another embodiment of the methods according to the present invention the one or more second antigenic molecules, which are provided with a labeling means, are provided prior to step (c) of the method of detecting autoantibodies according to the present invention.

In another embodiment of the methods according to the present invention the one or more first antigenic molecules, which are immobilized to a solid support and the one or more second antigenic molecules, which are provided with a first labeling means, are both provided prior to step (c) of the method of detecting autoantibodies according to the present invention.

In still other embodiments of the present invention the one or more first antigenic molecules are immobilized to a solid support and the one or more second antigenic molecules are provided with labeling means, whereby the one or more second antigenic molecules are provided in solution.

In yet other embodiments of the present invention the one or more first antigenic molecules are immobilized to a solid support and the one or more second antigenic molecules are provided with labeling means, whereby the one or more first antigenic molecules and the one or more second antigenic molecules are provided in solution.

Another methods of the present disclosure comprise directly monitoring the interaction of (i) analyte antibodies present in the sample and (ii) one or more first antigenic molecules and (iii) one or more second antigenic molecules and as provided by the present invention, by employing assay techniques substantially as known in the art (e.g., non-competitive or competitive assays), for example of the sandwich type or RET type, the latter assay type not requiring immobilization and separation of the one or more first antigenic molecules from the liquid phase.

Yet another methods of the present disclosure allow for the identification of modulators, which are capable to interact with the complexes formed in step (c) and/or which are capable to interfere with the complex formation according to step (c) as defined in the method of detecting analyte antibodies according to the present invention.

Accordingly, another methods of detecting analyte antibodies of the present disclosure further comprises step (f) prior to, or concurrent with, or subsequent to, step (c), providing one or more modulators capable to interact with the complexes formed in step (c) and/or capable to interfere with the complex formation according to step (c) and contacting said one or more modulators simultaneously or successively with the said sample, the said one or more first antigenic molecules, and/or the said one or more second antigenic molecules prior to, or concurrent with, or subsequent to step (c), or contacting said one or more modulators simultaneously or successively with the said complexes formed in or subsequent to step (c).

In another methods according to the present disclosure the one or more modulators are provided prior to step (c) of the method of detecting autoantibodies according to the present invention.

In still other embodiments of the methods according to the present invention one or more of the said means selected from the group consisting of the said immobilizing means and the said first labeling means are provided prior to step (c) of the method of detecting autoantibodies according to the present invention.

In a further aspect the present application discloses a kit, which is useful for the performance of any of the methods according to the present invention comprising (a) one or more first antigenic molecules selected from the IRF as defined in one or more of the methods according to the invention; (b) one or more second antigenic molecules selected from the IRF as defined in one or more of the methods according to the invention; (c₁) immobilization means as defined in one or more of the methods according to the invention; and (d) first labeling means as defined in one or more of the methods according to the invention, and, optionally, one or more analyte antibodies, which are reactive with the one or more first and second antigenic molecules as defined in one or more of the methods according to the invention. In another aspect the kit according to the present disclosure comprises (a) said first antigenic molecules immobilized to a solid support; and (b) said second antigenic molecules labeled with a first labeling means.

In still other embodiments the present invention provides the use of any of the methods according to the invention for the diagnosis of the presence or onset of a disease related to the insulin receptor family and/or for the identification of a pharmaceutically effective compound for the treatment and/or prophylaxis of a disease related to the insulin receptor family.

### Detailed Description of the Invention

Principally, the terms and phrases used in this application shall have the meaning of the general knowledge of the person skilled in the art. However, the following preferred meaning of specified terms and phrases may be noted:
The terms "polypeptide" and "protein" are used interchangeable throughout this application.

The term "sample" to be investigated according to the present invention shall preferably mean any sample which is essentially a liquid or suspension, preferably of biological and/or chemical origin. The sample can be obtained by well known techniques and may preferably mean isolated body fluids such as blood, plasma, serum, cerebrospinal fluid, saliva, urine, seminal liquid, tear fluids and others. The sample may further encompass nail clippings, faeces, other excrement, separated cells, cell homogenates, tissue homogenates, or organ homogenates obtained from an animal (e.g., mouse, rat, guinea pig, dog, pig, primates) or human individual. Tissue samples or organ samples may be obtained from any tissue or organ by, e.g., biopsy. Separated cells may be obtained from the body fluids or the tissues or organs by separating techniques such as centrifugation or cell sorting. Preferably, cell samples, tissue samples or organ samples are obtained from those cells, tissues or organs which express, contain, accumulate, concentrate or produce the antigenic molecules referred to herein. The sample may have been subject to treatment and/or modification, known to the person skilled in the art in order to allow storage or further processing in a method of the invention. For example, the person skilled in the art knows that the sample may be diluted in a suitable buffer, or, if the sample is derived from urine, any biotin contained in the sample need to be removed in order to avoid interference with accurate biotin determination, if biotin is used as a label means in the method of the invention. The sample may be selected from an individual suspected of the onset or presence of a dysfunctional condition selected from autoimmune disorders, growth, development or energy metabolism disorders, growth hormone binding protein disorder, growth hormone receptor disorder and growth hormone disorders. In one embodiment of the invention, the sample may comprise a known amount of analyte antibodies and/or one or more modulators, preferably, a known amount of both analyte antibodies and one or more modulator. This may be of particular relevance for the identification of modulators as hereinbefore described.

Generally, the term "antigenic molecule" according to the present invention means any molecule with which an analyte antibody can interact and which is capable of binding an (one or more) analyte antibody to form specific complexes comprising [analyte antibody-antigenic molecule]. The antigenic molecule may be natural or synthetic and modifications thereto are preferably such as to not detrimentally affect the binding properties in the methods according to the present invention.

The term "analyte antibodies" according to the present invention means any antibody capable of binding to any member of the IRF, respectively, capable of binding to one or more of the receptors selected from the group consisting of IR, IGF1R, IGF2R, IIHR, and IRRR as further outlined herein, whose presence is being quantitatively and/or qualitatively analyzed. The term "analyte antibody" may include a monoclonal antibody, a polyclonal antibody, a single chain antibody, a bispecific antibody or diabody, a bivalent antibody, a multispecific antibody, a synthetic antibody, an aptamer, a spiegelmer, a human or humanized antibody, and a fragment or variant thereof such as, e.g., Fab, Fv or scFv fragments, or a chemically modified derivative of any of these, e.g. antibody-drug conjugates, domain antibodies, nanobodies or antibody mimetica (DARPins, designed ankyrin repeat proteins). In specific embodiments of the present invention the term "analyte antibodies" means endogenous autoantibodies, therapeutic antibodies, and/or diagnostic antibodies.

The term "member of the insulin receptor family", respectively, "insulin receptor family" (IRF) according to the present invention means any polypeptide selected from the group consisting of the insulin receptor (IR), the insulin-like growth factor 1 receptor (IGF1R), the insulin-like growth factor 2 receptor (IGF2R), the insulin-IGF1 hybrid receptor (IIHR), the insulin receptor-related receptor (IRRR), any subunit, variant, analogue, derivative, and fragment thereof. Preferably, the IRF is of animal (e.g., mouse, rat, guinea pig, dog, pig, primates) or human origin, more preferred human.

In one embodiment of the methods of the invention the term "member of the insulin receptor family" or "insulin receptor family" (IRF) means the insulin receptor (IR) or any subunit, or any variant thereof. In another embodiment of the present invention only the said first antigenic molecules are selected from the insulin receptor (IR) or any subunit, or any variant thereof.

In a second embodiment of the methods of the invention the term "member of the insulin receptor family" or "insulin receptor family" (IRF) means the insulin-like growth factor 1 receptor (IGF1R) or any subunit, or any variant thereof. In another embodiment of the present invention only the said first antigenic molecules are selected from the insulin-like growth factor 1 receptor (IGF1R) or any subunit, or any variant thereof.

In a third embodiment of the methods of the invention the term "member of the insulin receptor family" or "insulin receptor family" (IRF) means the insulin-like growth factor 2 receptor (IGF2R) or any variant thereof. In another embodiment of the present invention only the said first antigenic molecules are selected from the insulin-like growth factor 2 receptor (IGF2R) or any variant thereof.

In a fourth embodiment of the methods of the invention the term "member of the insulin receptor family" or "insulin receptor family" (IRF) means the insulin-IGFl hybrid receptor (IIHR) or any subunit, or any variant thereof. In another embodiment of the present invention only the said first antigenic molecules are selected from the insulin-IGFl hybrid receptor (IIHR) or any subunit, or any variant thereof.

In a fifth embodiment of the methods of the invention the term "member of the insulin receptor family" or "insulin receptor family" (IRF) means the insulin receptor-related receptor (IRRR) or any subunit, or any variant thereof. In another embodiment of the present invention only the said first antigenic molecules are selected from the insulin receptor-related receptor (IRRR) or any subunit, or any variant thereof.

In a sixth embodiment of the methods of the invention the term "member of the insulin receptor family" or "insulin receptor family" (IRF) means any polypeptide selected from the insulin receptor (IR) or any subunit, or any variant thereof and the insulin-like growth factor 1 receptor (IGF1R) or any subunit, or any variant thereof.

In a seventh embodiment of the methods of the invention the term "member of the insulin receptor family" or "insulin receptor family" (IRF) means any polypeptide selected from the insulin receptor (IR) or any subunit, or any variant thereof and the insulin-like growth factor 2 receptor (IGF2R) or any variant thereof.

In an eighth embodiment of the methods of the invention the term "member of the insulin receptor family" or "insulin receptor family" (IRF) means any polypeptide selected from the insulin receptor (IR) or any subunit, or any variant thereof and the insulin-IGFl hybrid receptor (IIHR) or any subunit, or any variant thereof.

In a ninth embodiment of the methods of the invention the term "member of the insulin receptor family" or "insulin receptor family" (IRF) means any polypeptide selected from the insulin receptor (IR) or any subunit, or any variant thereof and the insulin receptor-related receptor (IRRR) or any subunit, or any variant thereof.

In a tenth embodiment of the methods of the invention the term "member of the insulin receptor family" or "insulin receptor family" (IRF) means any polypeptide selected from the insulin-like growth factor 1 receptor (IGF1R) or any subunit, or any variant thereof and the insulin-like growth factor 2 receptor (IGF2R) or any variant thereof.

In an eleventh embodiment of the methods of the invention the term "member of the insulin receptor family" or "insulin receptor family" (IRF) means any polypeptide selected from the insulin-like growth factor 1 receptor (IGF1R) or any subunit, or any variant thereof and the insulin-IGFl hybrid receptor (IIHR) or any subunit, or any variant thereof.

In a twelfth embodiment of the methods of the invention the term "member of the insulin receptor family" or "insulin receptor family" (IRF) means any polypeptide selected from the insulin-like growth factor 1 receptor (IGF1R) or any subunit, or any variant thereof and the insulin receptor-related receptor (IRRR) or any subunit, or any variant thereof.

In a thirteenth embodiment of the methods of the invention the term "member of the insulin receptor family" or "insulin receptor family" (IRF) means any polypeptide selected from the insulin-like growth factor 2 receptor (IGF2R) or any variant thereof and the insulin-IGFl hybrid receptor (IIHR) or any subunit, or any variant thereof.

In a fourteenth embodiment of the methods of the invention the term "member of the insulin receptor family" or "insulin receptor family" (IRF) means any polypeptide selected from the insulin-like growth factor 2 receptor (IGF2R) or any variant thereof and the insulin receptor-related receptor (IRRR) or any subunit, or any variant thereof.

In a fifteenth embodiment of the methods of the invention the term "member of the insulin receptor family" or "insulin receptor family" (IRF) means any polypeptide selected from the insulin-IGFl hybrid receptor (IIHR) or any subunit, or any variant thereof and the insulin receptor-related receptor (IRRR) or any subunit, or any variant thereof.

Suitable polypeptides and their corresponding genes, which encode the members of the IRF, are well known to the skilled person in the art. Members of the IRF are commercially available from recombinant sources (e.g. from R&D Systems, Inc., Minneapolis, MN 55413, USA, OriGene Technologies, Inc., Rockville, MD 20850, USA) and are well known from protein and nucleic acid sequence databases, such as, e.g., EMBL, Genbank and others. Currently available database accession numbers for members of the IRF are given for the human species at the specific receptor proteins, however, the present invention shall not be understood to be limited thereto.

The term "insulin receptor" (IR) according to the present invention means any isolated polypeptide having a naturally occurring amino acid sequence or any variant thereof. The amino acid sequences and gene sequences encoding IR are well known to the skilled person, e.g., from entries in sequence databases such as UniProtKB, e.g., P06213 (1382 amino acid length from *Homo sapiens*). In another embodiment of the present invention the IR is modified for use in in vitro analysis so that it does not comprise a functional intact tyrosine kinase domain or may completely lack the intracellular tyrosine kinase domain due to deletion, e.g., by use of recombinant technologies in protein modification, which are well known to the person skilled in the art. In still another embodiment of the present invention, IR means an IR variant, which exhibits pathogenic or dysfunctional prevalence in an animal (e.g., mouse, rat, guinea pig, dog, pig, primates) or human subject. In yet another embodiment of the present invention the IR is embedded in a membrane environment.

The term "insulin-like growth factor 1 receptor" (IGF1R) according to the present invention means any isolated polypeptide having a naturally occurring amino acid sequence or any variant thereof. The amino acid sequence and gene sequence encoding IGF1R are well known to the skilled person, e.g., from entries in sequence databases such as UniProtKB, e.g., P08069 (1367 amino acid length from *Homo sapiens*) or C9J5X1 (1366 amino acid length from *Homo sapiens*). In another embodiment of the present invention the IGF1R is modified for use in in vitro analysis so that it does not comprise a functional intact tyrosine kinase domain or may completely lack the intracellular tyrosine kinase domain due to deletion, e.g., by use of recombinant technologies in protein modification, which are well known to the person skilled in the art. In still another embodiment of the present invention, IGF1R means an IGF1R variant, which exhibits pathogenic or dysfunctional prevalence in an animal (e.g., mouse, rat, guinea pig, dog, pig, primates) or human subject. In yet another embodiment of the present invention the IGF1R is embedded in a membrane environment.

The term "insulin-like growth factor 2 receptor" (IGF2R) according to the present invention means any isolated polypeptide having a naturally occurring amino acid sequence or any variant thereof. The amino acid sequence and gene sequence encoding IGF2R are well known to the skilled person, e.g., from entries in sequence databases such as UniProtKB, e.g., P11717 (2491 amino acid length from *Homo sapiens*). In another embodiment of the present invention, IGF2R means an IGF2R variant, which exhibits pathogenic or dysfunctional prevalence in an animal (e.g., mouse, rat, guinea pig, dog, pig, primates) or human subject. In still another embodiment of the present invention the IGF2R is embedded in a membrane environment.

The term "insulin-IGF-1 hybrid receptor" (IIHR) according to the present invention means any isolated polypeptide having a naturally occurring amino acid sequence or any variant thereof. The amino acid sequence and gene sequence encoding IIHR are well known to the skilled person. In another embodiment of the present invention the IIHR is modified for use in in vitro analysis so that it does not comprise a functional intact tyrosine kinase domain or may completely lack the intracellular tyrosine kinase domain due to deletion, e.g., by use of recombinant technologies in protein modification, which are well known to the person skilled in the art. In still another embodiment of the present invention, IIHR means an IIHR variant, which exhibits pathogenic or dysfunctional prevalence in an animal (e.g., mouse, rat, guinea pig, dog, pig, primates) or human subject. In yet another embodiment of the present invention the IIHR is embedded in a membrane environment.

The term "insulin-receptor related receptor" (IRRR) according to the present invention means any isolated polypeptide having a naturally occurring amino acid sequence or any variant thereof. The amino acid sequence and gene sequence encoding IRRR are well known to the skilled person, e.g., from entries in sequence databases such as UniProtKB, e.g., P14616 (1297 amino acid length from *Homo sapiens*). In another embodiment of the present invention the IRRR is modified for use in in vitro analysis so that it does not comprise a functional intact tyrosine kinase domain or may completely lack the intracellular tyrosine kinase domain due to deletion, e.g., by use of recombinant technologies in protein modification, which are well known to the person skilled in the art. In still another embodiment of the present invention, IRRR means an IRRR variant, which exhibits pathogenic or dysfunctional prevalence in an animal (e.g., mouse, rat, guinea pig, dog, pig, primates) or human subject. In yet another embodiment of the present invention the IRRR is embedded in a membrane environment.

The term "variant" according to the present invention means any fragment, analog, derivative, fusion protein, subunit or subunit chain of a molecule mentioned. Preferably, the variant may have at least essentially the same biological properties as the respective polypeptides or proteins mentioned, except for the tyrosine kinase activity, immobilizing features and/or labeling features. Moreover, it is to be understood that the term "variant" according to the present invention shall include an amino acid sequence which differs due to at least one amino acid substitution, modification, deletion and/or addition, wherein the amino acid sequence of the variant is still, preferably, at least about 50%, at least about 60%, at least about 70%, at least about 80%, at least about 85%, at least about 90%, at least about 92%, at least about 95%, at least about 97%, at least about 98%, or at least about 99% identical with the amino sequence of the respective polypeptide or protein mentioned, preferably over the entire length of the specific polypeptide or protein. Variants may be allelic variants or any other species specific homologs, paralogs, or orthologs.

Moreover, the variants referred to herein include fragments of the respective polypeptides or proteins mentioned hereinbefore from the IRF, provided these fragments have essentially the same biological properties. Furthermore, the variants referred to herein include fusion proteins of the respective polypeptides or proteins mentioned hereinbefore from the IRF with polypeptides, which are suitable as immobilization means, labeling means, or label, provided such fusion protein essentially maintains the same biological properties of the respective polypeptide or protein mentioned hereinbefore from the IRF. Variants may further include modifications of the said polypeptides or proteins by glycosylation or any other chemical or enzymatic modification, provided these variants have essentially the same biological properties as referred to above.

The variants according to the invention may include so-called 'silent' substitutions, additions are deletions which do not alter or substantially alter the biological activity. More particularly, the variants according to the present invention may have been modified with respect to one or more of the amino acid residues, which are substituted by a conserved or non-conserved amino acid residue, preferably a conserved amino acid residue, or such ones in which one or more of the amino acid resides may include a substituted radical. Such variants are deemed to be within the scope of the teachings herein. Most typically, variants are those that vary by conservative amino acid substitutions. Such substitutions are those that substitute a given amino acid in a polypeptide by another amino acid of similar chemical characteristics. In this regard are understood as conservative substitutions the replacements, one for another, among the small aliphatic amino acids A, V, L and I; among the hydroxyl residues S and T; among the acidic residues D and E; among the amide residues N and Q; among the basic residues K and R; and among the aromatic residues F and Y (according to the single letter code of amino acids of the IUPAC nomenclature).

In one embodiment of the present invention, a "variant" shares essentially the same biological properties. In another embodiment of the present invention a "variant" of a member of the IRF is used, which exhibits pathogenic or dysfunctional prevalence, i.e., which exhibits a disease related to the insulin receptor family in an animal (e.g., mouse, rat, guinea pig, dog, pig, primates) or human subject.

According to another embodiment of the present invention the one or more members of the IRF are modified so that they do not comprise a functional intact tyrosine kinase domain or completely lack the intracellular tyrosine kinase domain, e.g., by means of recombinant technologies in protein modification, which are well known to the person skilled in the art.

The term "biological properties" in accordance with the understanding of the present invention means the binding properties of the respective molecule mentioned. In case of a specific member of the IRF it may be its ability to form a complex with insulin, IGF-1, and/or IGF-2 under suitable conditions. For the purpose of the present invention, it is understood that the signaling property of any member of the IRF with respect to the tyrosine kinase activity is expressly excluded. Optionally, the term "biological properties" may further include certain specific immunological properties of polypeptides, e.g., if they are specifically detectable by the same ELISA method. Particularly, a member of the IRF exhibits essentially the same "biological properties" like a variant thereof, if both (a) can interact with the analyte antibodies, (b) are detectable by the same ELISA method and/or (c) are detectable by the detection method according to the present invention.

In another preferred embodiment of the invention the one or more first antigenic molecules and the one or more second antigenic molecules are one or more polypeptides selected from the group consisting of IR, IGF1R, IGF2R, IIHR, and IRRR.

The term "providing prior to step (c) of the method of detecting autoantibodies", respectively, "providing prior to step (d) of the method for the identification of modulators" according to the present invention shall mean prior to contacting the said antigenic molecules and the said analyte antibodies in a method of the present invention.

The term "detecting the presence and/or the binding properties" according to the present invention means determining the amount or concentration, preferably quantitatively. The measuring can be done directly or indirectly. Direct measuring relates to measuring the amount or concentration of one or more of the reaction educts and/or the reaction products based on a signal which is obtained from the one or more reaction educts and/or the reaction products itself/themselves and the intensity of which directly correlates with the number of molecules of the one or more reaction educts and/or the reaction products in the reaction volume. Such a signal may be obtained, e.g., by measuring the intensity or value of a specific physical or chemical property of the one or more reaction educts and/or reaction products. Indirect measuring includes measuring of a signal obtained from a secondary component (i.e. a component not being the reaction educt or reaction product itself) or a biological read out system, referred to as "label means" in this specification, e.g., of measurable cellular or transmembrane responses, ligands, or enzymatic reaction products, e.g. by means of fluorophors, chromophors, ion concentrations, which suitably is performed by means of optical, electrical and/or electronical equipment. For measurement of enzymatic reaction products, preferably the amount of substrate is saturating. Optionally, the substrate may also be labeled with a detectable label prior to the reaction. Preferably, the reaction partners are contacted with the substrate for an adequate period of time, which corresponds to the time necessary for a detectable amount of the one or more reaction products to be produced such as a measurable signal. Instead of measuring the amount or concentration of the one or more reaction products, the time necessary for appearance of a given (e.g. detectable) amount or concentration of the one or more reaction products can be measured.

According to the present invention, "detecting the presence and/or the binding properties" can be achieved by all means for determining the amount of a reaction educt and/or reaction product known to the skilled person. Said means comprise immunoassay devices and methods which may utilize labeled molecules in various sandwich, competition, or other assay formats. Said assays will develop a signal which is indicative for the presence or absence of the reaction educts and/or the reaction products. Moreover, the signal strength can, preferably, be correlated directly or indirectly (e.g. reverse-proportional) to the amount of reaction educts and/or the reaction products in the reaction volume. Said methods comprise, preferably, biosensors, optical devices coupled to immunoassays, biochips, analytical devices such as spectrometers or chromatography devices. Further, methods include ELISA-based methods using, optionally pre-treated or pre-coated, micro-plates, micro-arrays, or tube-arrays, fully-automated or robotic immunoassays (available, e.g., on Roche-Elecsys™, Abbott-AxSYM™ or Brahms Kryptor™ analyzer systems). Preferably, "detecting the presence and/or the binding properties" comprises the steps which will allow bringing the reaction partners together for an adequate period of time.

The term "binding" according to the present invention includes both covalent and non-covalent binding. The term "specific binding" means a binding affinity of at least 3 times higher, preferably of at least about 10 times higher and more preferably of at least about 50 times higher than the binding affinity to other molecules. In another embodiment of the present invention the term "binding" shall mean the binding of binding partners in an in vitro binding assay under suitable conditions, preferably under conditions according to the assay manufacturer's instructions or according to the methods essentially as defined hereinafter in the Example section. Generally, "binding" means a binding affinity (K_{D} - which means the quotient of dissociation constant to association constant) of about 10⁻¹⁴ M to 10⁻⁷ M, preferably of about 10⁻¹³ M to 10⁻⁹ M.

A "modulator" according to the present disclosure includes any biological or chemical compound, a macromolecule (e.g. larger than about 5 kDa), a small molecule (e.g., smaller than about 5 kDa or even smaller than about 800 Da), an isolated compound or a mixture of compounds, an extract or homogenates of tissue or organ origin, or a compound of synthetic origin comprising, e.g., one or more peptides, polypeptides (e.g., polyclonal or monoclonal antibodies, including single chain antibodies, diabodies, multispecific antibodies, humanized antibodies, hybrid antibodies, or fragments thereof, such as Fv, Fab and F(ab)₂ fragments), aptamers, spiegelmers, nucleic acids, and/or small molecules.

The term "antigenic molecule embedded in a membrane environment" according to the present invention means that the said antigenic molecule - which is in case of the IRF a transmembrane protein - is provided in a model membrane or the like, which includes any suitable synthetic, natural or artificial environment of a ,e.g., cellular, membrane, vesicular, micellar or liposomal structure, whereby the said antigenic molecule is able to interact with the aforementioned analyte antibodies and thus complex formation.

Assay methods for proteins embedded in a membrane environment are well known to the skilled person, e.g., from technologies and assays which use artificial or biomimetical membranes or lipid bilayers (herein "model membranes"). Model membranes are widely used for investigating membrane proteins and many of them are suitable for the performance of the methods of the present invention, particularly, if denaturation of the said antigenic molecule can be avoided.

The term "model membrane" according to the present invention encompasses, e.g., any liposome or vesicle, such as artificially prepared vesicles comprising one, two or more lipid layers in spherical geometry. Such liposomes and vesicles are used as vehicles for the transport of lipids, proteins and small molecules; therefore they are used in the administration of pharmaceuticals. Vesicles or liposomes can easily be prepared by disrupting biological membranes from cells cultures, tissues, organs, or subcellular structures (e.g. nucleus, Golgi, endoplasmatic reticulum, and mitochondria), e.g., by sonication and/or extrusion) and subsequent self-reassembling of the lipid structures, whereby labeling with dyes or labeled polypeptides suitable in RET is easily achievable. Further "model membranes" may comprise lipid bilayers which have been synthetically assembled in vitro. They can be made from one or more synthetic and/or natural lipids.

The term "model membranes" according to the present invention may further include, e.g., black lipid membranes (BLM), vesicles, lipid bilayers, liposomes, micelles, bicelles, hybrid bilayers (e.g. comprising a hydrophobic monolayer and a lipid monolayer), and nanodiscs, which may be anchored, supported or tethered to a solid phase or solid substrate, and which may, optionally, be provided with a spacer or cushion (e.g., polyethylene glycols, oligonucleotides, peptides, polypeptides (e.g., streptavidin), hydrogels and others) which may allow to maintain a distance of the membrane and/or the aforementioned antigenic molecule to the solid substrate. Preferably, the spacer or cushion is a hydrophilic molecule. In contrast to a vesicle or a cell membrane, the aforementioned supported bilayer may have a planar structure sitting on a solid support. Therefore, only the upper face of the bilayer is exposed to the solution. For example, the preparation of proteoliposomes is well known from European patent application EP 1992688 A1, of which the liposome preparation methods as disclosed in any of examples 1 to 20 are herewith incorporated by reference.

The term "micelles" according to the present invention means another type of model membranes which lack a lipid bilayer. In aqueous solutions, micelles are assemblies of amphipathic molecules (e.g., detergents) with their hydrophilic heads exposed to solvent and their hydrophobic tails in the center. Micelles are able to solubilize membrane proteins by partially encapsulating them and shielding their hydrophobic surfaces from solvent. The term "bicelles" means still another type of model membranes which are typically made of two lipids, one of which forms a lipid bilayer while the other forms an amphipathic, micelle-like assembly shielding the bilayer center from surround solvent molecules. The term "nanodiscs" means a segment of a bilayer encapsulated by an amphipathic protein coat, a lipid or detergent layer. Membrane proteins can be incorporated into and solubilized by nanodiscs.

The term "disease related to the insulin receptor family" according to the present invention preferably means any dysfunction which is related to one or more polypeptides selected from the group consisting of IR, IGF1R, IGF2R, IIHR, and IRRR, more preferably of one, two, or three selected polypeptides of the said IRF. The term "disease related to the insulin receptor family" according to the present invention encompasses autoimmune disorders (such as, e.g., insulin dependent diabetes mellitus (IDDM), Morbus Basedow, Graves Orbitopathy) and also disorders and dysfunctions such as obesity, neurological disorders, growth disorders, cancer generation and development (including breast, colon, ovarian, prostate, lung), and other cellular proliferative disorders and dysfunctions, whether of autoimmune origin or not, preferably of autoimmune origin.

The term "immobilizing means" according to the present invention means any reagent and/or process, which is suitable to immobilize the said first antigenic molecules to a solid support according to the knowledge of the person skilled in the art. With respect to the kind of a solid support and conditions employed in accordance with the present invention, the solid support and conditions do generally not fundamentally differ from conventionally used solid supports and conditions employed in known immunoassay techniques. A solid support for use according to the present invention can comprise an ELISA plate as currently employed in known ELISA techniques, or may employ any other suitable support for use in the present invention, such as micro-titer plates (having 96, 384, 1536, or 3456 wells or more) or parts thereof, tubes, particles, magnetic beads, nitrocellulose or the like. Materials suitable as solid support which can be used in accordance with the teachings of the present invention are well known in the art and include, e.g., commercially available column materials, polystyrene beads and other carriers, latex beads, magnetic beads, colloidal metal, glass surfaces and chips for use in protein microchip technologies, silanylated surfaces and chips for use in protein microchip technologies, silicon surfaces and chips for use in protein microchip technologies, nitrocellulose carriers, cellulose carriers, membranes, model membranes, stabilized liposomes or cells (e.g., duracytes), wells and walls of reaction trays or microtiter plates, plastic tubes etc.

The antigenic molecules as hereinbefore defined may be immobilized to any carrier which is known to the person skilled in the art. Examples of such carriers include glass, polystyrene, polyvinyl chloride, polypropylene, polyethylene, polycarbonate, dextran, nylon, amylose, natural and modified cellulose, polyacrylamide, agarose, magnetite, and gold. The carrier can be either soluble or insoluble, in case of an insoluble carrier; the carrier is a solid or colloid and may, optionally, be provided as suspension.

Suitable methods for immobilizing said antigenic molecules are well known and include, but are not limited to ionic, hydrophobic, covalent interactions and the like. It may also be suitable to use solid immobilization means in suspension according to the present invention, wherein the carrier is provided in suspension, e.g. a microbead or microsphere consisting of different microbeads or microspheres, optionally labeled, both carrying each different molecules. Methods of producing such suspensions, e.g., based on solid-phase chemistry and photo-labile protective groups, are, e.g., known from US 5,744,305.

The term "labeling" according to the present invention means labeling by direct or indirect methods. Direct labeling involves coupling of the label directly (covalently or non-covalently) to the molecule to be labeled. Indirect labeling involves binding (covalently or non-covalently) of a second ligand to the molecule to be labeled. Such second ligand should specifically bind to the molecule to be labeled with an at least 3-fold higher, preferably at least 10-fold, and more preferred at least 50-fold higher affinity under assay conditions. Said second ligand may be coupled with a suitable label means and/or may bind a third ligand binding to the second ligand. The use of second, third, or even higher order ligands is often used to increase the signal. Suitable second and higher order ligands may include antibodies, secondary antibodies, and the well-known streptavidin-biotin system (Vector Laboratories, Inc.). Furthermore, the molecule to be labeled or the substrate may also be "tagged" with one or more tags known in the art. Such tags may then be targets for higher order ligands. Suitable tags include biotin, digoxygenin, His-Tag, Glutathion-S-Transferase, FLAG-tag (N-DYKDDDDK-C), green fluorescence protein (GFP), myc-tag, influenza a virus haemagglutinin (HA), maltose binding protein, and others. In the case of a peptide or polypeptide, the tag is generally located at or close to the N-terminus and/or C-terminus.

Furthermore, the molecule to be labeled or the substrate may also be provided with a suitable "spacer" known in the art in order to avoid in case of bulky molecules any limitations with respect to the binding properties due to spatial constrictions.

The term "first labeling means" according to the invention shall preferably mean any direct or indirect detectable labeling means selected from the group of enzymatic labels, isotopic or radioactive labels, chemoluminescent labels, bioluminescent labels, fluorescent labels, magnetic labels (e.g. "magnetic beads", including paramagnetic and superparamagnetic labels), dye labels (chromophors), and others known in the art. Suitable labels are detectable by an appropriate detection method known in the art. Suitable labels may further include gold particles, latex beads, acridan ester, luminol, and ruthenium. Preferably suitable are non-radioactive labels.

Enzymatically active labels include, e.g., horseradish peroxidase, alkaline phosphatase, beta-galactosidase, luciferase, and derivatives thereof. Suitable substrates for detection include di-amino-benzidine (DAB), 3, 3'-5, 5'-tetramethylbenzidine, NBT-BCIP (4-nitro blue tetrazolium chloride and 5-bromo-4-chloro-3-indolyl-phosphate, CDP-Star™ (Amersham Biosciences), ECL™ (Amersham Biosciences) and others known in the art.

A suitable enzyme-substrate combination may result in increase or decrease of a colored reaction product (chromophor), fluorescence, or chemo- or bioluminescence, which can be measured according to methods known in the art (e.g. using a photometer, a photo-multiplier, and a light-sensitive film or camera system). The same principles apply for measuring the endpoint or performance or development of an enzymatic reaction.

Suitable fluorescence labels include fluorescent dyes and proteins (such as GFP and its derivatives), Cy3, Cy5, Texas Red, fluorescein, and the Alexa dyes (e.g. Alexa 568). Further suitable fluorescent labels are commercially available e.g. from Molecular Probes (Oregon, USA). Also the use of quantum dots as fluorescent labels is encompassed. Examples of fluorescent proteins include, but are not limited to, green, yellow, cyan, blue, and red fluorescent proteins.

Suitable chemoluminescence or bioluminescence labels include, but are not limited to prokaryotic (e.g., bacterial lux-encoded) or eukaryotic (e.g., firefly luc-encoded) luciferases, as well as variants possessing varied or altered optical properties, such as luciferases that produce different colors of light, e.g. derived from *Photinus pyralis,* from the sponge *Suberities domuncula,* and the *Mycena* fungi. Furthermore, photoproteins, e.g., calcium-activated photoproteins and their specifically designed variants may be suitable, which are capable of producing light typically in the range of 200 nm to 1100 nm, or in the visible spectrum (i.e., between approximately 350 nm and 800 nm), e.g., obelin from the marine polyp *Obelia longissima,* or Aequorin, e.g., from the luminescent jellyfish *Aequorea victoria* or from other organisms may be suitable, optionally in a membrane.

Suitable radioactive labels include ³⁵S, ¹²⁵I, ³²P, ³³P and the like. A radioactive label can be detected by any method known and appropriate, e.g. a light-sensitive film or a phosphor imager.

Suitable detection methods according to the present invention also include precipitation (particularly immunoprecipitation), electrochemiluminescence (electrically generated chemiluminescence), biolominescence, RIA (radioimmunoassay), ELISA (enzyme-linked immunosorbent assay), sandwich enzyme immune tests, sandwich immunoassays (ECLIA), dissociation-enhanced lanthanide fluorescent Immunoassay (DELFIA™, PerkinElmer Inc., USA), scintillation proximity assay (SPA), turbidimetry, nephelometry, latex-enhanced turbidimetry or nephelometry, latex agglutination assay, or solid phase immune assays.

Further methods known in the art (such as gel electrophoresis, 2D gel electrophoresis, SDS polyacrylamid gel electrophoresis (SDS-PAGE), Western Blotting, and mass spectrometry), can optionally be used in combination with the labeling or other detection methods as described above.

The one or more antigenic molecules according to the invention may be directly or indirectly provided with the labeling means, substantially as hereinbefore described.

### Sequences:

Seq. ID No. 1 Primer P1 (33mer)
Seq. ID No. 2 Primer P2 (34mer)
Seq. ID No. 3 Primer P3 (35mer)
Seq. ID No. 4 Primer P4 (33mer)
Seq. ID No. 5 DNA encoding amino acids 2-551 from firefly luciferase
Seq. ID No. 6 amino acid sequence 2-551 from firefly luciferase
Seq. ID No. 7 DNA encoding amino acids 1-1367 from human IGF1R
Seq. ID No. 8 amino acid sequence 1-1367 from human IGF1R
Seq. ID No. 9 DNA encoding amino acids 1-1919 from human IGF1R-luc fusion
Seq. ID No. 10 amino acid sequence 1-1919 from human IGF1R-luc fusion
Seq. ID No. 11 DNA encoding amino acids 1-1370 from human insulin receptor
Seq. ID No. 12 amino acid sequence 1-1370 from human insulin receptor (IR)
Seq. ID No. 13 DNA encoding amino acids 1-1922 from human IR-luc fusion
Seq. ID No. 14 amino acid sequence 1-1922 from human insulin receptor-luc fusion
Seq. ID No. 15 DNA encoding amino acids 1-927 from the extracellular domain of human IGF1R (ECDhIGF1R)
Seq. ID No. 16 amino acid sequence 1-927 from the extracellular domain of human IGF1R (ECDhIGF1R)
Seq. ID No. 17 Primer P5 (33mer)
Seq. ID No. 18 Primer P6 (33mer)
Seq. ID No. 19 Primer P7 (36mer)
Seq. ID No. 20 Primer P8 (83mer)

The present invention will now be illustrated by the following examples, which shall be understood not to limit the scope of the present invention in any way.

### Experimental Procedures:

### Materials:

DNA primers (P1-P8) were obtained from BioTeZ Berlin Buch GmbH (Berlin, Germany); pSP-luc+NF vector was obtained from Promega GmbH (Mannheim, Germany); pIRESneo vector was obtained from Clontech (Palo Alto, CA, USA); vector pCR-XL-TOPO-IR was obtained from ImaGenes GmbH (Berlin, Germany); vector pFastBac1 was obtained from Invitrogen; Goat anti human IgG (SIGMA) was labeled with Acridinium NHS Ester (Cayman Chemical Company, Ann Arbor, MI, USA); IGF1 was obtained from Acris Antibodies (Herford, Germany); Insulin was obtained from Sigma-Aldrich; anti-IGFIR (Tyrl 165/Tyrl 166) antibody was obtained from Novus Biologicals (Edinburgh, UK); CellTiter-Glo luminescent cell viability assay kit was obtained from Promega GmbH; High Five insect cells were purchased from Invitrogen; Polystyrene tubes coated with PGA14 antibody (Selenotest LIA) were obtained from ICI immunochemical intelligence GmbH (Berlin, Germany). If not otherwise stated, all other reagents and chemicals were obtained from Sigma-Aldrich Chemie GmbH (Munich, Germany) or Merck KGaA (Darmstadt, Germany); enzymes were obtained from Promega or New England Biolabs (Ipswich, MA, USA).

### Example 1: Construction of fusion proteins

### Example 1A: Construction of an IGF1R-luciferase fusion protein

The DNA (Seq. ID No. 5) encoding amino acids 2-551 from firefly luciferase (Seq. ID No. 6 on pSP-luc+NF) was amplified by PCR using primers P1 (Seq. ID No. 1) and P2 (Seq. ID No. 2) containing EcoRI and BamHI restriction sites, respectively. pIRESneo was digested with EcoRI and BamHI restriction endonucleases; the obtained fragment was replaced with the DNA encoding firefly luciferase obtained from the aforementioned PCR resulting in plasmid pIRESneo-Luc.

The DNA (Seq. ID No. 7) encoding amino acids 1-1367 from human IGF1R (Seq. ID No. 8) was amplified by PCR using primers P3 (Seq. ID No. 3) and P4 (Seq. ID No. 4) containing NotI and EcoRI restriction sites, respectively. pIRESneo-Luc was digested with NotI and BamHI restriction endonucleases and the obtained fragment was replaced with the DNA sequence encoding human IGF1R obtained from the aforementioned PCR resulting in vector pIRESneo-IGF1R-Luc containing Seq. ID No. 9 encoding Seq. ID No. 10.

### Example 1B: Construction of an IR-luciferase fusion protein

The DNA (Seq. ID No. 11 of pCR-XL-TOPO-IR) encoding amino acids 1-1370 from human IR (Seq. ID No. 12) was amplified by PCR using primers P5 (Seq. ID No. 17) and P6 (Seq. ID No. 18) containing HpaI and MfeI restriction sites, respectively. pIRESneo-Luc was digested with EcoRV and EcoRI restriction endonucleases and the obtained fragment was replaced with the DNA sequence encoding human IR obtained from the aforementioned PCR resulting in vector pIRESneo-IR-Luc containing Seq. ID No. 13 encoding Seq. ID No. 14.

Example 1C: Construction of a fusion protein of the extracellular domain of IGF1R (ECDhIGFIR) and the 16 amino acid epitope recognized by PGA14 antibodies The DNA (Seq. ID No. 15) encoding 927 amino acids of the extracellular domain of the human IGF1 receptor (Seq. ID No. 16, ECDhIGFIR) was amplified by PCR using primers P7 (Seq. ID No. 19) and P8 (Seq. ID No. 20) containing BamHI and HindIII restriction sites, respectively (P8 containing the coding sequence for the 16 amino acid epitope recognized by PGA14 antibodies). pFastBac1 vector was digested with BamHI and HindIII restriction endonucleases and the obtained fragment was replaced with the DNA sequence encoding the ECD of hIGFIR obtained from the aforementioned PCR resulting in vector pFastBac1-IGF1R-PGA14tag.

### Example 2: Generation of IGF1R-Luc and IR-Luc fusion protein producing cells

### Example 2A: Generation of IGF1R-Luc and IR-Luc producing HEK 293

HEK 293 cells were grown in DMEM supplemented with 10% fetal bovine serum. Cells were cultivated in a 5% CO2 atmosphere at 37° C. HEK 293 cells were transfected with pIRESneo-IGF1R-Luc vector or pIRESneo-IR-Luc vector using FuGENE6 transfection reagent (obtained from Roche Deutschland Holding GmbH, Grenzach-Wyhlen, Germany) according to the manufacturer's instruction. 48 hours after transfection, selection was started with 0.8 mg/ml G418 (Gibco™ BRL, Invitrogen). Stable clones expressing high levels of fusion protein were selected.

### Example 2B: Generation of recombinant baculovirus expressing ECDhIGF1R-PGA14tag fusion protein

The ECDhIGF1R-PGA14tag sequence obtained from example 1C was transferred to bacmid DNA by site-specific recombination in bacteria. The bacmid was then used to generate a fully recombinant baculovirus in Sf9 insect cells according to the protocols supplied by the manufacturer (Bac-to-Bac expression system manual, Invitrogen).

### Example 2C: Production of IGF1R-PGA14tag fusion protein

Suspension High Five insect cells were grown in Express Five serum-free medium to density 2x10e6 cells/ml. Cells were then infected with recombinant IGF1R-PGA14tag-baculovirus at a multiplicity of infection (MOI) of 1. 72 hours post-infection cell medium was collected and stored at -80C°.

### Example 3: Control of expression products

### Example 3A: Interaction of IGF1R-Luc cells with IGF1

Confluent HEK 293 cells grown in a 96 well plate were washed with DMEM/F12 medium and incubated with acridinium labeled IGF1 (about 1.0e06 RLU/well) diluted in 100 µl of DMEM/F12, 0.1% BSA for 3 h at 37° C in a 5% CO₂ atmosphere. In further experiments unlabelled IGF1 (0.3 mg/ml) was added together with acridinium labeled IGF1 to determine unspecific binding. After incubation cells were washed with DMEM, resuspended in PBS containing 2% triton X-100 and obtained lysates were measured in a luminometer Berthold Technologie AutoLumat Plus LB 953 for 10 sec. Each value represents the mean value (RLU) +/- standard deviation (SD) of duplicate measurements (relative light units (RLU) x 1000).

| **Table 1** | **Wildtype HEK 293** | **IGF1R-Luc HEK 293** |
|---|---|---|
| Labeled IGF1 | 27 +/- 4 | 55 +/- 7 |
| Labeled IGF1 + excess IGF1 | 11 +/- 1 | 12 +/- 1 |

This result indicates that IGF1R-luc fusion proteins are correctly processed and expressed at the cell membrane.

### Example 3B: Interaction of IR-Luc cells with insulin

Confluent HEK 293 cells grown in a 96 well plate were washed with DMEM/F12 medium and incubated with acridinium labeled insulin about 1.0e06 RLU/well) diluted in 100 µl of DMEM/F12, 0.1% BSA for 3 h at 37° C in a 5% CO₂ atmosphere. In further experiments unlabelled insulin (0.3 mg/ml) was added together with acridinium labeled insulin to determine unspecific binding. After incubation the cells were washed with DMEM, resuspended in PBS containing 2% triton X-100 and obtained lysates were measured in a luminometer Berthold Technologie AutoLumat Plus LB 953 for 10 sec. Each value represents the mean value (RLU) +/- standard deviation (SD) of duplicate measurements (RLU x 1000).

| **Table 2** | **Wild type HEK 293** | **IR-Luc HEK 293** |
|---|---|---|
| Labeled insulin | 32 +/- 2 | 78 +/- 6 |
| Labeled insulin + excess insulin | 13 +/- 2 | 17 +/- 3 |

This result indicates that IR-luc fusion proteins are correctly processed and expressed at the cell membrane.

### Example 4: Preparation of IGF1R-Luc and IR-Luc cell extract

Confluent HEK 293 cells (producing either IGF1R-Luc or IR-Luc) grown in a 75 cm² plate were resuspended by scraping into PBS and were washed in the same buffer by centrifugation at 2500 rpm. The resulting cells were lysed in 0.5 ml buffer containing 20 mM HEPES-NaOH pH 7.5, 50 mM NaCl, 1% Triton X-100, 10% glycerol. The suspension was centrifuged at 5,000 rpm for 15 min and the supernatant was collected and stored at -80 C.

### Example 5: Immunoprecipitation assay for IGF1R autoantibodies

The IGF1R-Luc cell extract was diluted 10 times with buffer containing 20 mM HEPES-NaOH pH 7.5, 50 mM NaCl, 1% Triton X-100, 10% glycerol, 5 mg/ml BSA. For immunoprecipitation, 100 µl of diluted extract (about 1.0e07 RLU) was mixed with 10 µl of a sample (serum probe) and incubated overnight at 4°C. Immune complexes were precipitated by addition of 100 µl of 10% protein A-sepharose suspension in same buffer for 1 h at room temperature with shaking. Protein A-sepharose was pelleted and washed 3 times with 1 ml of washing buffer (10 mM Tris-HCl, pH 7.5, 60 mM NaCl, 0.02 % Tween 20). Finally, luciferase activity was measured in a Berthold luminometer (AutoLumat Plus LB 953) for 10 sec. Results were expressed as RLU bound. Each value represents the mean value of duplicate measurements. The ability of crude sera and isolated IgG preparations from the same sera IgG to immunoprecipitate the IGF1 receptor was compared.

| **Table 3** | **Serum [RLU]** | **RLU-serum [% of max]** | **IgG [RLU]** | **RLU-IgG [% of max]** |
|---|---|---|---|---|
| 1 | 417 | 0 | 238 | 0 |
| 2 | 619 | 0 | 181 | 0 |
| 3 | 781 | 1 | 392 | 1 |
| 4 | 669 | 0 | 414 | 1 |
| 5 | 348 | 0 | 293 | 0 |
| 6 | 121585 | 83 | 70472 | 92 |
| 7 | 145972 | 100 | 76178 | 100 |
| 8 | 130683 | 90 | 62441 | 82 |
| 9 | 55840 | 38 | 23002 | 30 |
| 10 | 131560 | 90 | 49699 | 65 |

### Table 4: Dilution, recovery and stability of the IGF1R autoantibody assay.

(A) Five different sera containing autoantibodies were diluted with incubation buffer and measured in assay as described in example 5. (B) Five sera with IGF1R autoantibodies, five sera without IGF1R autoantibodies and their mixtures (1:1) were analyzed in assay as described in example 5. (C) Mix of five sera was incubated at room temperature (RT) and 4°C for given periods of time and analyzed in IGF1R autoantibodies assay as described in example 5.

### Table 4A: Dilution experiments for the IGF1R autoantibodies assay.

Five different sera containing autoantibodies were diluted with incubation buffer and measured in assay as described in example 5. Bound luciferase activity was measured as previously described. Each value represents the mean value of duplicate measurements (RLU x 1000).

| **Table 4A Sera (%)** | **Serum 1** | **Serum 2** | **Serum 3** | **Serum 4** | **Serum 5** |
|---|---|---|---|---|---|
| 100 | 76,3 | 93,7 | 71,8 | 41,3 | 86,6 |
| 50 | 50,2 | 54,6 | 39,6 | 21,5 | 51,8 |
| 25 | 27,1 | 30,1 | 24,2 | 13,9 | 27,6 |
| 10 | 11,6 | 13,9 | 11,4 | 6,6 | 12,2 |

### Table 4B: Recovery experiments for the IGF1R autoantibodies assay.

Five sera without IGF1R autoantibodies (A), five sera with IGF1R autoantibodies (B) and their mixtures (1:1) were analyzed in the assay as described in example 5. Bound luciferase activity was measured as previously described. Each value represents the mean value of duplicate measurements (RLU x 1000).

| **Table 4B** | **1** | **2** | **3** | **4** | **5** |
|---|---|---|---|---|---|
| Serum A | 4,9 | 5,6 | 3,3 | 3,2 | 3,4 |
| Serum B | 63,3 | 76,1 | 60,3 | 25,4 | 63,2 |
| Mix A+B | 35,0 | 42,1 | 33,4 | 15,0 | 37,4 |

### Table 4C: Stability experiments for the IGF1R autoantibodies assay.

Serum with IGF1R autoantibodies was incubated for different time at 4°C and at room temperature (RT). Serum samples were analyzed in assay as described in example 5. Bound luciferase activity was measured as previously described. Each value represents the mean value of duplicate measurements (RLU x 1000).

| **Table 4C** | **0 days** | **5 days** | **10 days** |
|---|---|---|---|
| 4°C | 53,0 | 52,5 | 49,4 |
| RT | 53,0 | 52,2 | 53,8 |

### Example 6: Isolation of human IgG

1 ml of human serum was mixed with 1 ml PBS and 0.2 ml of protein G-sepharose and incubated overnight at 4°C by shaking. Protein G-sepharose was pelleted and washed ten times with PBS. Bound IgG were eluted with 25 mM citric acid, pH was adjusted to 7 using 1M Hepes-NaOH, pH 8. Eluted IgG were concentrated to 100 µl using Speedvac at room temperature. IgG was transferred into DMEM/F12 medium using speed filtration on sephadex G25 column.

### Example 7: Effect of autoantibodies on IGF1R autophosphorylation

HepG2 cells were seeded in 96 well plates (10 000 cells/well), incubated 24 h in complete DMEM/F12 medium followed by overnight incubation in serum free DMEM/F12 medium containing 0.1% BSA. After serum starvation cells were incubated with human IgG (10-20 mg/ml) in DMEM/F12 medium for 15 min up to 1h. After this in some cases IGF1 (1 ng/ml) was added and cells were incubated for the additional 15 min. Cells were washed with PBS containing phosphatase inhibitors and lysed in buffer 20 mM HEPES-NaOH pH 7.5, 50 mM NaCl, 2% triton X100, 10% glycerol, phosphatase inhibitors. Cell lysates were subjected to electrophoresis in 10% SDS PAGE and blotted to nitrocellulose membranes. Tyrosine phosphorylated IGF1R was detected on the blot using an anti-IGFIR (Tyrl 165/Tyr1166) antibody. Bands were visualized using enhanced chemiluminescence western blotting detection kit (Amersham ECL Plus, GE Healthcare, General Electric Deutschland Holding GmbH, Frankfurt, Germany).

### Example 8: Effect of autoantibodies on cell growth

MCF7 cells were seeded at 2500 cells per well (96 well plates) and incubated overnight in complete DMEM/F12 medium. Cells were then starved in serum free DMEM/F12 medium containing 0.1% BSA for 5 h followed by addition of IgG (about 10 mg/ml) from five sera with low (1-5) and five sera with high (6-10) concentrations of autoantibodies against IGF1R (isolated from human patients), 1% FCS and 1 ng/ml IGF1. Cells were incubated for 5 days. The CellTiter-Glo luminescent cell viability kit was used to assess the number of viable cells in culture based on quantification of the ATP present, according to the manufacturer manual. Inhibiting effect of autoantibodies was expressed as Inhibition Index calculated as InI % = 100 x [(1 - (RLU test IgG) / (RLU negative IgG pool)]. Each value represents the mean value of duplicate measurements.

| **Table 5** | **Serum** | **Inhibition Index (%)** |
|---|---|---|
| | 1 | -7 |
| | 2 | 1 |
| | 3 | -3 |
| | 4 | -3 |
| | 5 | -2 |
| | 6 | 20 |
| | 7 | 18 |
| | 8 | 22 |
| | 9 | 16 |
| | 10 | 21 |

### Example 9: Bridge assays

### Example 9A: Bridge assay for the detection of autoantibodies interacting both with IGF1 receptor and insulin receptor.

Polystyrene tubes coated with PGA14 antibody were incubated overnight at 4°C with 200 µl of SF6 insect cell medium containing IGF1R-PGA14tag. After IGF1R-PGA14tag immobilization tubes were washed twice with 1ml of buffer 20 mM Tris-HCl pH 7.5, 50 mM NaCl, 10 % glycerol. Then each tube was incubated overnight at 4C° with a mixture of 100 µl of the same buffer containing 10 mg/ml BSA and 100 µl of a sample (serum probe). Tubes were washed twice with 1ml of the same buffer and incubated overnight at 4C° with 200 µl of IGF1R-Luc (or IR-Luc respectively) diluted in the same buffer with BSA (about 40x 10e6 RLU of luciferase activity). After incubation tubes were washed four times with 1ml of 20 mM Tris-HCl pH 7.5, 100 mM NaCl, 0.1 % triton X100. Finally, luciferase activity was measured in a Berthold luminometer (AutoLumat Plus LB 953) for 10 sec. Results were expressed as RLU (relative light units) bound. Each value represents the mean value of duplicate measurements.

| **Table 6 Bridge Assay** | **IGF1R-Luc [RLU]** | **IGF1R-Luc [% of max]** | **IR-Luc [RLU]** | **IR-Luc [% of max]** |
|---|---|---|---|---|
| without serum | 2135 | 5 | 947 | 5 |
| control serum | 5348 | 13 | 2616 | 15 |
| serum 406 | 42240 | 100 | 4814 | 27 |
| serum 522 | 39341 | 93 | 17961 | 100 |
| serum 531 | 36646 | 87 | 12839 | 71 |

### Example 9B: Detection limits of patient serum

An IGF1R-Ab positive serum was diluted with 20 mM Tris-HCl pH 7.5, 50 mM NaCl, 10% glycerol, 10 mg/ml BSA. The assay was performed as described in Example 9A. Negative serum is defined to give a signal like buffer control.

| **Table 7** | **Serum dilution (v/v)** | **IGF1R-Luc [RLU]** |
|---|---|---|
| | Undiluted | 64651 |
| | 1 / 4 | 38829 |
| | 1 / 16 | 20269 |
| | 1 / 64 | 8135 |
| | 1 / 256 | 5383 |
| | background (negative serum) | 5369 |

### Example 9C: Detection limits of commercially available anti-IGFIR antibodies

Monoclonal antibody to the extracellular domain of IGF1R (anti-IGFIR clone#24-75, Millipore, USA) was diluted with 20 mM Tris-HCl pH 7.5, 50 mM NaCl, 10% glycerol, 10 mg/ml BSA. The assay was performed as described in Example 9A.

| **Table 8 Antibody concentration (ng/ml)** | **IGF1R-Luc [RLU]** |
|---|---|
| 50000 | 805261 |
| 12500 | 767157 |
| 3125 | 626879 |
| 781 | 475770 |
| 195 | 234773 |
| 49 | 88894 |
| 12 | 27013 |
| 3 | 11831 |
| 0.8 | 8856 |
| 0.2 | 5904 |
| 0.05 | 5369 |
| background | 5418 |

This result indicates that the detection limit of the assay according to the invention is about 0.2 ng/ml antibody concentration.

### Example 10: Clinical Relevance of the autoantibody level against IRF member protein in humans.

Examination of serum samples of 1001 probands (healthy adult humans) were analysed for the presence of insulin receptor autoantibody levels. A prevalence of 10% positive individuals (n= 103) was determined comparing autoantibody positive and negative individuals revealing a significant difference in basal glucose concentrations (p=0.03).

## Claims

1. A method for the measurement of autoantibodies to IRF members as serological markers comprising
detecting in a sample to be investigated the presence and/or the binding properties of analyte antibodies reactive with one or more antigenic molecules,
said method comprising:
(a) providing one or more first antigenic molecules with which analyte antibodies when present in said sample can interact and which first antigenic molecule is selected from the insulin receptor family (IRF); and
(b) providing one or more second antigenic molecules with which analyte antibodies when present in said sample can interact and which second antigenic molecule is selected from the IRF; and
(c) contacting said first antigenic molecules as provided by step (a) and said second antigenic molecules as provided by step (b) simultaneously or successively with the sample to be investigated, whereby analyte antibodies when present in said sample can interact with said antigenic molecules so as to form complexes comprising [first antigenic molecule]-[analyte antibody]-[second antigenic molecule]; and
(d) prior to, or concurrent with, or subsequent to, step (c), providing immobilizing means whereby said first antigenic molecule as present in the said complexes formed in step (c), respectively, as capable to form complexes in step (c) is immobilized to a solid support prior to, or concurrent with, or subsequent to, step (c); and
(e) prior to, or concurrent with, or subsequent to, step (c), providing first labeling means whereby said second antigenic molecule as present in the said complexes formed in step (c), respectively, as capable to form complexes in step (c) is labeled with said first labeling means prior to, or concurrent with, or subsequent to, step (c); and
(g) detecting the presence of said complexes formed in or subsequent to step (c) so as to provide indication of analyte antibodies present in said sample.

2. The method according to claim 1, further comprising:
(f) prior to, or concurrent with, or subsequent to, step (c), providing one or more modulators capable to interact with the complexes formed in step (c) and/or capable to interfere with the complex formation according to step (c) and contacting said one or more modulators simultaneously or successively with the said sample, the said one or more first antigenic molecules, and/or the said one or more second antigenic molecules prior to, or concurrent with, or subsequent to step (c), or contacting said one or more modulators simultaneously or successively with the said complexes formed in or subsequent to step (c).

3. The method according to one or more of claims 1 to 2, wherein said first antigenic molecules and said second antigenic molecules are identical.

4. The method according to one or more of claims 1 to 3, wherein said first antigenic molecules and/or said second antigenic molecules are embedded in a membrane environment.

5. The method according to one or more of claims 1 to 4, wherein said one or more first antigenic molecules and/or said one or more second antigenic molecules are lacking a functionally intact tyrosine kinase domain.

6. The method according to one or more of claims 1 to 5, wherein the said analyte antibody to be detected in said sample is an endogenous autoantibody or a monoclonal antibody.

7. The method according to one or more of claims 1 to 5, wherein one or more of the said means selected from the group consisting of said first labeling means, said immobilization means, and said modulators are provided prior to the contacting said antigenic molecules and said analyte antibodies.

8. The use of the method according to one or more of claims 1 to 7 for the diagnosis of the presence or onset of a disease related to the insulin receptor family.

9. The use of the method according to one or more of claims 1 to 7 for the identification of a pharmaceutically effective compound for the treatment and/or prophylaxis of a disease related to the insulin receptor family.

## Patentansprüche

1. Verfahren zur Messung von Autoantikörpern gegen IRF-Mitglieder als serologische Marker umfassend
den Nachweis der Anwesenheit und/oder von Bindungseigenschaften von Analytantikörpern in einer zu untersuchenden Probe, die mit einem oder mehreren antigenen Molekülen reagieren, besagte Methode umfassend:
(a) Bereitstellen von einem oder mehreren ersten antigenen Molekülen, die mit Analytantikörpern interagieren können, sofern in besagter Probe anwesend, und wobei das erste antigene Molekül aus der Insulin Rezeptor Familie (IRF) ausgewählt ist; und
(b) Bereitstellen von einem oder mehreren zweiten antigenen Molekülen, die mit Analytantikörpern interagieren können, sofern in besagter Probe anwesend, und wobei das zweite antigene Molekül aus der IRF ausgewählt ist; und
(c) Kontaktieren von besagten ersten antigenen Molekülen wie in Schritt (a) bereitgestellt und besagten zweiten antigenen Molekülen wie in Schritt (b) bereitgestellt, gleichzeitig oder nacheinander mit der zu untersuchenden Probe, wobei Analytantikörper, sofern in besagter Probe anwesend, mit besagten antigenen Molekülen interagieren können, so dass Komplexe gebildet werden umfassend [erstes antigenes Molekül]-[Analytantikörper]-[zweites antigenes Molekül]; und
(d) das vorherige, gleichzeitige oder zu Schritt (c) nachfolgende Bereitstellen von Immobilisierungsmitteln, wobei besagtes erstes antigenes Molekül wie in den in Schritt (c) gebildeten Komplexen anwesend, bzw. wie in Schritt (c) fähig, Komplexe zu bilden, vorher, gleichzeitig oder nachfolgend zu Schritt (c) an einen festen Träger immobilisiert wird; und
(e) das vorherige, gleichzeitige oder zu Schritt (c) nachfolgende Bereitstellen von Markierungsmitteln, wobei besagtes zweites antigenes Molekül wie in den in Schritt (c) gebildeten Komplexen anwesend, bzw. wie in Schritt (c) fähig, Komplexe zu bilden, vorher, gleichzeitig oder nachfolgend zu Schritt (c) mit besagten Markierungsmitteln markiert wird; und
(g) Nachweis der Anwesenheit der besagten Komplexe, die in Schritt (c) oder nachfolgend gebildet wurden, zur Feststellung der Anwesenheit der Analytantikörper in besagter Probe.

2. Das Verfahren nach Anspruch 1, weiterhin umfassend:
(f) das vorherige, gleichzeitige oder zu Schritt (c) nachfolgende Bereitstellen von einem oder mehreren Modulatoren, welche fähig sind, mit den in Schritt (c) gebildeten Komplexen zu interagieren und/oder fähig sind, mit der Komplexbildung gemäß Schritt (c) zu interferieren und das gleichzeitige oder nachfolgende Kontaktieren von besagten einem oder mehreren Modulatoren mit besagter Probe, besagten einem oder mehreren ersten antigenen Molekülen, und/oder besagten einem oder mehreren zweiten antigenen Molekülen vorher, gleichzeitig oder nachfolgend zu Schritt (c), oder das Kontaktieren von besagten einem oder mehreren Modulatoren gleichzeitig oder nachfolgend mit besagten Komplexen, die in Schritt (c) oder nachfolgend gebildet wurden.

3. Das Verfahren nach einem oder mehreren der Ansprüche 1 bis 2, worin besagte erste antigene Moleküle und besagte zweite antigene Moleküle identisch sind.

4. Das Verfahren nach einem oder mehreren der Ansprüche 1 bis 3, worin besagte erste antigene Moleküle und/oder besagte zweite antigene Moleküle in einer Membranumgebung eingebettet sind.

5. Das Verfahren nach einem oder mehreren der Ansprüche 1 bis 4, worin besagten einen oder mehreren ersten antigenen Molekülen und/oder besagten einen oder mehreren zweiten antigenen Molekülen eine funktional intakte Tyrosinkinase-Domäne fehlt.

6. Das Verfahren nach einem oder mehreren der Ansprüche 1 bis 5, worin besagter Analytantikörper, der in besagter Probe nachzuweisen ist, ein endogener Autoantikörper oder ein monoklonaler Antikörper ist.

7. Das Verfahren nach einem oder mehreren der Ansprüche 1 bis 5, worin ein oder mehrere der besagten Mittel ausgewählt aus der Gruppe bestehend aus den ersten Markierungsmitteln, den Immobilisierungsmitteln und den Modulatoren vor dem Kontaktieren von besagten antigenen Moleküle und besagten Analytantikörpem bereitgestellt werden.

8. Die Verwendung eines Verfahrens nach einem oder mehreren der Ansprüche 1 bis 7 zur Diagnose der Anwesenheit oder des Beginns einer Erkrankung, die in Bezug zu der Insulin Rezeptor Familie steht.

9. Die Verwendung eines Verfahrens nach einem oder mehreren der Ansprüche 1 bis 7 zur Identifikation einer pharmazeutisch wirksamen Verbindung zur Behandlung und/oder Prophylaxe einer Erkrankung, die in Bezug zu der Insulin Rezeptor Familie steht.

## Revendications

1. Procédé de mesure d'anticorps contre des membres de la famille des récepteurs de l'insuline IRF servant de marqueurs sérologiques, comprenant
la détection, dans un échantillon à examiner, de la présence, et/ou des propriétés de liaison d'anticorps analyte réactifs avec une ou plusieurs molécules antigéniques,
ledit procédé comprenant :
(a) la fourniture d'une ou de plusieurs premières molécules antigéniques avec lesquelles les anticorps analyte, lorsqu'ils sont présents dans ledit échantillon, peuvent interagir, et laquelle première molécule antigénique est choisie dans la famille des récepteurs de l'insuline (IRF) ; et
(b) la fourniture d'une ou de plusieurs secondes molécules antigéniques avec lesquelles les anticorps analyte, lorsqu'ils sont présents dans ledit échantillon, peuvent interagir, et laquelle seconde molécule d'antigène est choisie dans l'IRF ; et
(c) la mise en contact desdites premières molécules antigéniques telles que fournies par l'étape (a) et desdites secondes molécules antigéniques telles que fournies par l'étape (b), simultanément ou successivement, avec l'échantillon à examiner, ce par quoi, les anticorps analyte, lorsqu'ils sont présents dans ledit échantillon, peuvent interagir avec lesdites molécules antigéniques de sorte à former des complexes comprenant [première molécule antigénique] - [anticorps analyte] - [seconde molécule antigénique] ; et
(d) avant l'étape (c), ou de manière concomitante ou subséquente à celle-ci, la fourniture de moyens d'immobilisation, ce par quoi, ladite première molécule antigénique, lorsqu'elle est présente dans lesdits complexes formés dans l'étape (c), respectivement, lorsqu'elle est capable de former des complexes dans l'étape (c), est immobilisée sur un support solide avant l'étape (c), ou de manière concomitante ou subséquente à celle-ci ; et
(e) avant l'étape (c), ou de manière concomitante ou subséquente à celle-ci, la fourniture de premiers moyens de marquage, ce par quoi, ladite seconde molécule antigénique, lorsqu'elle est présente dans lesdits complexes formés dans l'étape (c), respectivement, lorsqu'elle est capable de former des complexes dans l'étape (c), est marquée avec lesdits premiers moyens de marquage avant l'étape (c), ou de manière concomitante ou subséquente à celle-ci ; et
(g) la détection de la présence desdits complexes formés pendant l'étape (c) ou de manière subséquente à celle-ci de sorte à fournir une indication d'anticorps analytes présents dans ledit échantillon.

2. Procédé selon la revendication 1, comprenant en outre :
(f) avant l'étape (c), ou de manière concomitante ou subséquente à celle-ci, la fourniture d'un ou de plusieurs modulateurs capables d'interagir avec les complexes formés dans l'étape (c) et/ou capables d'interférer avec la formation de complexe selon l'étape (c), et la mise en contact dudit ou desdits modulateurs, simultanément ou successivement, avec ledit échantillon, ladite ou lesdites premières molécules antigéniques, et/ou ladite ou lesdites secondes molécules antigéniques avant l'étape (c) ou de manière concomitante ou subséquente celle-ci, ou la mise en contact dudit ou desdits modulateurs simultanément ou successivement avec lesdits complexes formés dans l'étape (c) ou de manière subséquente à celle-ci.

3. Procédé selon une ou plusieurs des revendications 1 à 2, dans lequel lesdites premières molécules antigéniques et lesdites secondes molécules antigéniques sont identiques.

4. Procédé selon une ou plusieurs des revendications 1 à 3, dans lequel lesdites premières molécules antigéniques et/ou lesdites secondes molécules antigéniques sont incorporées dans un environnement membranaire.

5. Procédé selon une ou plusieurs des revendications 1 à 4, dans lequel ladite ou lesdites premières molécules antigéniques et/ou ladite ou lesdites secondes molécules antigéniques sont dépourvues d'un domaine de tyrosine kinase fonctionnellement intact.

6. Procédé selon une ou plusieurs des revendications 1 à 5, dans lequel ledit anticorps analyte a détecter dans ledit échantillon est un anticorps endogène ou un anticorps monoclonal.

7. Procédé selon une ou plusieurs des revendications 1 à 5, dans lequel un ou plusieurs desdits moyens choisis dans le groupe constitué desdits premiers moyens de marquage, desdits moyens d'immobilisation et desdits modulateurs sont fournis avant la mise en contact desdites molécules antigéniques et desdits anticorps d'analytes.

8. Utilisation du procédé selon une ou plusieurs des revendications 1 à 7 pour le diagnostic de la présence ou d'une apparition d'une maladie liée à la famille des récepteurs d'insuline.

9. Utilisation du procédé selon une ou plusieurs des revendications 1 à 7 pour l'identification d'un composé pharmaceutiquement efficace pour le traitement et/ou la prophylaxie d'une maladie liée à la famille des récepteurs de l'insuline.
